# EUROPÄISCHE PATENTSCHRIFT

(11) **EP 2 077 322 B1**
(45) Veröffentlichungstag und Bekanntmachung des Hinweises auf die Patenterteilung: **25.08.2010**
(21) Anmeldenummer: 09155974.0
(22) Anmeldetag: 25.07.2006
(51) Int. Cl.: C12N 9/12, C12Q 1/68

(54) **Verfahren und Kit zur Amplifikation von heteropolymerer oder poly(C)- RNA**
Method and kit for amplifying heteropolymeric or poly(C) RNA
Procédé et kit d'amplification de ARN hétéropolymérique ou poly(C)

(30) Priorität: 25.07.2005 DE 102005036085; 13.02.2006 DE 102006008219
(43) Veröffentlichungstag der Anmeldung: 08.07.2009
(62) Teilanmeldung aus: 06761834.8
(73) Patentinhaber: RiboxX GmbH, 01445 Radebeul (DE)
(72) Erfinder: Rohayem, Jacques, 01309, Dresden (DE); Jäger, Katrin, 01462, Dresden (DE); Jacobs, Enno, 01309, Dresden (DE); Rudolph, Wolfram, 01307, Dresden (DE)
(74) Vertreter: Habermann, Gert

(56) Entgegenhaltungen:
- WO-A2-02/092774
- SEAH E.L. ET AL.,: "open reading frame 1 of the norwalk-like virus camberwell: completion of sequence and expression in mammalian cells" J. VIROLOGY, Bd. 73, Nr. 12, Dezember 1999 (1999-12), Seiten 10531-10535, XP002418059
- DATABASE UNITPROT [Online] ID: Q9WI83_9CALI 1. November 1999 (1999-11-01), XP002418062 gefunden im EBI Database accession no. Q9WI83
- WANG J. ET AL.,: "sequence diversity of small, round-structured viruses in the norwalk virus group" J. VIROLOGY, Bd. 68, Nr. 9, August 1994 (1994-08), Seiten 5982-5990, XP002418060
- DATABASE WPI Week 200534 Thomson Scientific, London, GB; AN 2005-326212 XP002418065 -& JP 2005 110517 A (BML KK) 28. April 2005 (2005-04-28)
- GUO M. ET AL.,: "molecular characterization of porcine enteric calicivirus genetically related to sapporo-like human caliciviruses" J. VIROLOGY, Bd. 73, Nr. 11, November 1999 (1999-11), Seiten 9625-9631, XP002418061
- DATABASE UniProt [Online] ID: Q1W764_9CALI 2. Mai 2006 (2006-05-02), BEHM A. ET AL.,: "generation and molecular characterization of a feline calicivirus full-length clone" XP002537760 gefunden im EBI Database accession no. Q1W764
- DATABASE UniProt [Online] ID: POLG_SVM93 1. November 1997 (1997-11-01), XP002537761 gefunden im EBI Database accession no. Q69014
- DATABASE PDB [Online] 24. April 2006 (2006-04-24), XP002537762 gefunden im NCBI Database accession no. 2CKW_A

## Beschreibung

Die Erfindung betrifft ein Verfahren und ein Kit zur Primer-unabhängigen Amplifikation von Ribonukleinsäure (RNA) gemäß Anspruch 1 bzw. 7. Das erfindungsgemäße Amplifikationsverfahren eignet sich insbesondere für die Microarray-Technologie, zur Herstellung von siRNA und zur Diagnose viraler Infektionen durch den Nachweis viraler RNA in Patientenmaterial.

Die RNA ist ein wichtiger Bestandteil der eukaryontischen und der prokaryontischen Zelle. Sie ist an mehreren vitalen Funktionen beteiligt: der Transkription, d.h. der Umschreibung der Information des Erbgutes (Desoxyribonukleinsäure, DNA), der Übertragung dieser Information vom Zellkern in das Zytoplasma (mRNA), und der Translation, d.h. die Übersetzung der umgeschriebenen Information in Aminosäuren bzw. Proteine. Die RNA bildet auch die sog. Transfer-RNA, einen wichtigen Baustein für die Translation. Die RNA ist auch ein wichtiger Bestandteil der Ribosomen. Dies sind strukturelle Einheiten des Zytoplasmas, an denen die Übersetzung der Information in Eiweiß stattfindet.

Die Bedeutung der RNA im Bereich der Biotechnologie hat in den letzten Jahrzehnten zugenommen. Neue Entwicklungen wie z.B. die Microarray-Technologie, die miRNA (micro-RNA) oder die siRNA-Technologie (small interfering RNA) haben stattgefunden, deren Relevanz im Bereich der Grundlagenforschung als auch der angewandten Forschung zunimmt. All diese Technologien beruhen auf der synthetischen Generierung von RNA durch Amplifikation *in vitro.*

Die Herstellung von siRNA *in vitro* erfolgt bislang über chemische Synthese oder mit Hilfe von DNA-abhängigen RNA-Polymerasen wie der T7-RNA-Polymerase. Dafür werden zwei komplementäre DNA-Stränge mittels PCR unter Einsatz eines Primers, der auch die Sequenz für einen T7-Promotor enthält, synthetisiert. Nach Umschreiben der DNA mit Hilfe der T7 RNA-Polymerase werden die resultierenden komplementären RNA-Stränge hybridisiert und mittels RNAse 1 verdaut.

Im medizinischen Bereich spielt die RNA-Amplifikation eine wichtige Rolle beim Nachweis viraler Infektionen im Patientenmaterial. Weltweit werden 80% aller viralen Infektionen durch RNA-Viren, d.h. virale Erreger mit einem aus RNA bestehenden Erbgut, verursacht. Wichtige Erreger aus dieser Gruppe von Viren sind u.a. das Humane Immundefizienz-Virus (HIV), das Hepatitis-C-Virus (HCV), das Hepatitis-A-Virus, das Grippe-Virus (Influenza A, B und C) aber auch das Vogelpest-Virus, das vor kurzem neu identifizierte SARS-Virus, das Virus der Kinderlähmung (Pollovirus), das Masern-Virus, das Mumps-Virus, das Röteln-Virus und die Brechdurchfall-Viren (Rotavirus, Sapovirus und Norovirus).

Der Nachweis von RNA im Patientenmaterial findet bis dato in vier Schritten statt. Nach der Gewinnung der RNA aus dem Untersuchungsmaterial folgen die Schritte:
1. Umschreibung der RNA in copy-DNA (cDNA) durch RNA-abhängige DNA-Polymerasen, die sog. reversen Transkriptasen. Dafür bedarf es auch des Einsatzes eines Primers. Primer sind Oligonukleotide, die spezifisch für die zu amplifizierende Sequenz sind. Primer und die RNA-abhängige DNA-Polymerase ermöglichen die gezielte Amplifikation vom Erbgut, allerdings in DNA-Form.
2. Amplifikation der cDNA durch Polymerasekettenreaktion (PCR),
3. Aufreinigung des PCR-Produkts,
4. Sequenzierung des PCR-Produkts.

Für die Herstellung von RNA, beispielsweise zum Expressionsnachweis oder zur Herstellung von siRNA, erfolgt zusätzlich die Umschreibung des PCR-Produkts in RNA durch DNA-abhängige RNA-Polymerasen, wie z.B. die T7-Polymerase.
Die Durchführung dieser Schritte dauert in der Regel 18 bis 24 Stunden. Ebenfalls dazu benötigt wird auch ein gewisses "Know-How", was nicht allen Forschungseinrichtungen zugänglich ist.

Eine weitere Methode zur Amplifikation von RNA ist die Replikation durch RNA-abhängige RNA-Polymerasen wie z.B. die RNA-Polymerase des Bakteriophagen Qβ (auch Qβ - Replikase genannt), die RNA-Polymerase des Poliovirus oder des Hepatitis-C-Virus.

Das Genom des Bakteriophagen Qβ besteht aus einer einzelsträngigen (+)-RNA, die durch die RNA-abhängige RNA-Polymerase repliziert wird. Die Qβ - Replikase stellt eine (-)-Strang-RNA-Kopie des (+)-Stranges her, welche ebenso wie (+)-Stränge als Matrize fungieren kann. Auf diese Weise kann eine exponentielle Vervielfältigung erreicht werden. Das Qβ -System wird bereits zum Nachweis von Analyten, wie etwa Nukleinsäuren, verwendet (Chu et al. (1986), Nucleic Acids Research 14, 5591 - 5603; Lizardi et al. (1988), Biotechnology 6, 1197 - 1202).

Diese RNA-abhängigen RNA-Polymerasen benötigen jedoch bestimmte RNA-Sequenzen zur Initiation der RNA-Replikation. Zudem sind sie für die Amplikation der RNA auch auf bestimmte Hilfsproteine angewiesen. Daher sind diese RNA-abhängigen RNA-Polymerasen nicht dazu in der Lage, heterologe RNA zu amplifizieren.

Die WO 02/092774 A2 beschreibt ein Verfahren zu Amplifikation von RNA mit Hilfe eines Oligonukleotid-Primers, der eine so genannte SLC (stem-loop-cytosine)-Struktur aufweist. Der Erfindung liegt daher die Aufgabe zugrunde, ein Verfahren und Kits zur Amplifikation von viraler, eukaryontischer und prokaryontischer einzel- und doppelsträngiger Ribonukleinsäure (RNA) bereitzustellen, die effizienter und schneller sind.

Erfindungsgemäß wird die Aufgabe gelöst durch ein Verfahren zur Amplifikation von heteropolymerer RNA oder von poly(C)-RNA gemäß Anspruch 1, wobei die RNA-abhängige RNA-Polymerase vorzugsweise eine RNA-abhängigie RNA-Polymerase eines Virus ist, der zu den Viren der Familie der *Caliciviridae* gehört, und eine "Recht-Hand-Konformation" aufweist und die Aminosäuresequenz der RNA-abhängigen RNA-Polymerase die folgenden Sequenzabschnitte umfasst:
a. XXDYS
b. GXPSG
c. YGDD
d. XXYGL
e. XXXXFLXRXX
mit der Bedeutung:
D: Aspartat
Y: Tyrosin
S: Serin
G: Glycin
P: Prolin
L: Leucin
F: Phenylalanin
R: Arginin
X: eine beliebige Aminosäure.

Unter einer sog. "Rechte-Hand-Konformation" ist dabei zu verstehen, dass die tertiäre Struktur (Konformation) des Proteins eine Faltung nach einer rechten Hand mit Finger (finger), Handfläche (palm) und Daumen (thumb), aufweist.

Bei dem Sequenzabschnitt "XXDYS" handelt es sich um das sog. A-Motiv. Das A-Motiv ist für die Diskriminierng zwischen Ribonukleosiden und Deoxyribonukleosiden zuständig.
Bei dem Sequenzabschnitt "GXPSG" handelt es sich um das sog. B-Motiv. Das B-Motiv ist in allen Vertretern der Familie *Caliciviridae* konserviert.
Bei dem Sequenzabschnitt "YGDD" handelt es sich um das sog. C-Motiv. Das C-Motiv stellt die aktive Stelle des Enzyms dar. Dieses Motiv spielt bei der Koordinierung der Metallionen eine wichtige Rolle. Bei dem Sequenzabschnitt "XXYGL" handelt es sich um das sog. D-Motiv. Das D- Motiv Ist ein Merkmal der Template-abhängigen Polymerasen.

Bei dem Sequenzabschnitt "XXXXFLXRXX" handelt es sich um das sog. E-Motiv. Das E-Motiv ist ein Merkmal der RNA-abhängigen RNA Polymerasen, und kommt bei DNA-abhängigen RNA-Polymerasen nicht vor.

Die erfindungsgemäß verwendete RNA-abhängige RNA-Polyemerase verfügt u.a. über die folgenden Funktionen:
- Mg²⁺- oder Mn²⁺-abhängige RNA-abhängige RNA-Polymeraseaktivität, keine DNA-Abhängigkeit
- Template-Abhängigkeit
- primer-unabhängige RNA-Synthese mit poly(C)-RNA als Matrize in Anwesenheit von erhöhten GTP-Konzentrationen
- Primer-unabhängige RNA-Synthese mit heteropolymerer RNA als Matrize.

Die Primer-unabhängige Amplifikation der RNA erfolgt in der Abwesenheit eines Sequenzspezifischen Primers. Die Amplifikation ist Sequenz-unabhängig und erfolgt durch Einbau von AMP, GMP, CMP oder UMP.

Überraschenderweise ist die erfindungsgemäß verwendete RNA-abhängige RNA-Polymerase in der Lage, *in vitro* heterologe virale, eukaryontische und prokaryontische heteropolymer RNA als Template für die Primer-unabhängige Amplifikationsreaktion zu nutzen. Sowohl positiv-strängige und negativ-strängige einzelsträngige als auch doppelsträngige RNA ist als Template zur Primer-unabhängigen Amplifikation einsetzbar.

Vorzugsweise ist die erfindungsgemäß verwendete RNA-abhängige RNA-Polymerase eine RNA-abhängige RNA-Polymerase eines Virus aus der Familie der *Caliciviridae.* Das Genom der *Caliciviridae* besteht aus einem polyadenylierten (+)-strängigen RNA-Einzelstrang. *In vivo* schreibt die RNA-abhängige RNA-Polymerase des Calicivirus bei der viralen Replikation die genomische Calicivirus-RNA in eine (-)-strängige antisense-RNA (aRNA) um. Dabei entsteht ein RNA-aRNA-Hybrid. Die (-)-strängige aRNA dient im Anschluß als Templat zur Synthese neuer (+)-strängiger genomischer Virus-RNA.

In weiteren bevorzugten Ausführungsformen der Erfindung ist die RNA-abhängige RNA-Polymerase eine RNA-abhängige RNA-Polymerase eines humanen und / oder nicht-human pathogenen Calicivirus. Besonders bevorzugt handelt es sich um eine RNA-abhängige RNA-Polymerase eines Norovirus, Sapovirus, Vesivirus oder eines Lagovirus, z.B. die RNA-abhängige RNA-Polymerase des Norovirus-Stammes HuCV/NL/Dresden174/1997/GE oder des Sapovirus-Stammes pJG-Sap01 oder des Vesivirus-Stammes FCV/Dresden/2006/GE.

In besonders bevorzugten Ausführungsformen der Erfindung ist die RNA-abhängige RNA-Polymerase ein Protein mit einer Aminosäuresequenz gemäß **SEQ ID NO 1, SEQ ID NO 2** oder **SEQ ID NO 3**.

**SEQ ID NO 1** ist die Aminosäuresequenz einer Norovirus-RdRP.
**SEQ ID NO 2** ist die Aminosäuresequenz einer Sapovirus-RdRP.
S**EQ ID NO 3** ist die Aminosäuresequenz einer Vesivirus-RdRP.

Die RNA-abhängigen RNA-Polymerasen des Norovirus, des Sapovirus und des Vesivivirus sind rekombinant herstellbar durch Klonierung der die Norovirus-, Sapovirus- bzw. Vesivirus-RdRP kodierenden Nukleinsäure in einen geeigneten Expressionsvektor, beispielsweise pET-28 (Novagen). Der Expressionsvektor mit der Gensequenz, die für die Virus-RdRP kodiert, wird zur Expression in einen geeigneten Wirtsorganismus eingebracht. Der Wirtsorganismus wird bevorzugt aus den üblicherweise für die Proteinexpression verwendeten prokaryontischen, bevorzugt *Eschericha coli,* oder eukaryontischen Wirtsorganismen, bevorzugt *Sacharomyces cerevisae* oder Insektenzellen (bevorzugt Sf9-Zellen), die mit rekombinanten Baculoviren infiziert worden sind, gewählt. Dieser Wirtsorganismus enthält mindestens einen Expressionsvektor mit einer Gensequenz, die für die Virus-RdRP kodiert.

In bevorzugten Ausführungsformen wird die Norovirus-, Sapovirus- oder Vesivirus- Calicivirus-RdRP am N- oder C-Terminus mit einer Proteinsequenz fusioniert, weiche die Aufreinigung des RdRP-Fusionsproteins nach der Expression in einem Wirtsorganismus erleichtert. Bevorzugt ist diese Sequenz ein sogenannter Histidin-Marker, bestehend aus einer Sequenz von mindestens 6 aufeinanderfolgenden Histidinen (His oder H). Ein solcher Histidin-Marker ermöglicht in bekannter Weise die Aufreinigung des Proteins affinitätschromatographisch auf einer Nickel- oder Kobalt-Säule.

Beispiele für mit einem Histidin-Marker fusionierte Ausführungsformen der RNA-abhängigen RNA-Polymerase sind die Proteine mit einer Aminosäuresequenz gemäß **SEQ ID NO 4, SEQ ID NO 5** oder **SEQ ID NO 6.**

**SEQ ID NO 4** ist die Aminosäuresequenz einer Norovirus-RdRP mit einem Histidin-Marker (His-tag).
**SEQ ID NO 5** ist die Aminosäuresequenz einer Sapovirus-RdRP mit einem Histidin-Marker (His-tag).
**SEQ ID NO 6** ist die Aminosäuresequenz einer Vesivirus-RdRP mit einem Histidin-Marker (His-tag).

Die vorliegende Erfindung bezieht sich auf ein Verfahren zur Primer-unabhänigen Amplifikation von heteropolymerer oder poly(C)-RNA mittels einer RNA-abhängigen RNA-Polyermerase gemäß der Definition im Anspruch 1 mit den Schritten
a. Anlagerung der RNA-abhängigen RNA-Polymerase an die RNA-Matrize in der Abwesenheit eines Oligonukleotid-Primers,
b. Umschreiben der RNA-Matrize in antisense-RNA durch die RNA-abhängige RNA-Polymerase,
c. Trennung des RNA/antisense-RNA-Duplexes in einzelne Stränge durch Hitzedenaturierung, chemische Denaturierung und/oder durch ein Enzym mit der Fähigkeit, doppelsträngige RNA in einzelsträngige RNA aufzutrennen
sowie ein Kit zur Durchführung des Verfahrens zur Primer-unabhängigen Amplifikation von heteropolymerer oder poly(C)-RNA, mindestens bestehend aus
a. einer RNA-abhängigen RNA-Polyermase gemäß der Definition in Anspruch 1
b. einem geeigneten Reaktions-Puffer
c. NTPs
d. einem Enzym mit der Fähigkeit, doppelsträngige RNA in einzelsträngige RNA aufzutrennen
e. ggf. RNase-Inhibitor
f. ggf. Stoplösung.

Die RNA-Matrize wird beim erfindungsgemäßen Verfahren vorzugsweise in Mengen von 1 µg bis 4 µg pro 50 µl-Reaktionsansatz eingesetzt. Die Konzentration der eingesetzten Ribonukleotide ATP, CTP, GTP und UTP (NTPs) beträgt vorzugsweise zwischen 0,1 µmol/l und 1 µmol/l, besonders bevorzugt 0,4 µmol/l. Auch NTP-Analoga sind beim erfindungsgemäßen Verfahren zur RNA-Amplifikation einsetzbar, beispielsweise fluoreszenzmarkierte NTPs, Biotin-markierte NTPs oder radioaktiv markierte NTPs wie [P³²]-markierte NTPs. Die Konzentration der erfindungsgemäß verwendeten RdRP beträgt bevorzugt zwischen 1 µmol/l und 3µmol/l.

In einer besonders bevorzugten Ausführungsform enthält das Kit
a. 150 µmol/l rekombinant hergestellter Norovirus-RdRP gemäß **SEQ ID NO 1** oder **SEQ ID NO 4** und/oder Sapovirus-RdRP gemäß **SEQ ID NO 2** oder **SEQ ID NO 5** und/oder Vesivirus-RdRP gemäß **SEQ ID NO 3** oder **SEQ ID NO 6**
b. als Puffer: 50 mmol/l HEPES, pH 8.0, 3 mmol/l Magnesiumacetat oder Manganchlorid (MnCl₂), 4 mM DTT
c. 10 mmol/l ATP, 10 mmol/l CTP, 10 mmol/l GTP, 10 mmol/l UTP
d. ein Enzym mit der Fähigkeit, doppelsträngige RNA in einzelsträngige RNA aufzutrennen
e. RNase-Inhibitor
f. als Stoplösung: 4 mol/l Ammoniumacetat, 100 mmol/l EDTA.

Das Verfahren wird vorzugsweise bei einem pH-Wert zwischen 6,5 und 8,5 und bei einer Temperatur zwischen 20°C und 40°C durchgeführt. Eine bevorzugte Ausführungsform des Verfahrens zur RNA-Amplifikation wird bei 30°C mit Norovirus-RdRP oder bei 37°C mit Sapovirus-RdRP unter folgenden Pufferbedingungen durchgeführt: 50 mmol/l HEPES, pH 8.0, 3 mmol/l Magnesiumacetat oder Manganchlorid, 4 mmol/l DTT.

Erfindungsgemäß erfolgt die Trennung des RNA / antisense-RNA-Duplexes in einzelne RNA-Stränge durch Hitzedenaturierung, chemische Denaturierung und/oder mit einem Enzym mit der Fähigkeit, doppelsträngige RNA in einzelsträngige RNA aufzutrennen wie z.B. eine Helikase.

Durch die Strangtrennung liegt wieder einzelsträngiges Template vor und eine weitere Amplifikationsrunde kann eingeleitet werden.
Durch den Einsatz eines Enzyms mit der Fähigkeit, doppelsträngige RNA in einzelsträngige RNA aufzutrennen, kann die Reaktion isotherm gehalten werden.

Bestandteil der Erfindung ist ein entsprechendes Kit zur Primer-unabhängigen Amplifikation von heteropolymerer oder poly(C)-RNA, das ein Enzym mit der Fähigkeit, doppelsträngige RNA in einzelsträngige RNA aufzutrennen wie z.B. eine Helikase, enthält.

Die Erfindung betrifft ein Verfahren gemäß Anspruch 1 zur Primer-unabhängigen Amplifikation von heteropolymerer oder poly(C)-RNA, wobei kein Primer, der mit einem Abschnitt der RNA-Matrize hybridisiert, eingesetzt und durch die RNA-abhängige RNA-Polymerase entsprechend der Sequenz der RNA-Matrize verlängert wird, sowie ein Kit zur Primer-unabhängigen Amplifikation von heteropolymerer oder poly(C)-RNA gemäß Anspruch 7.

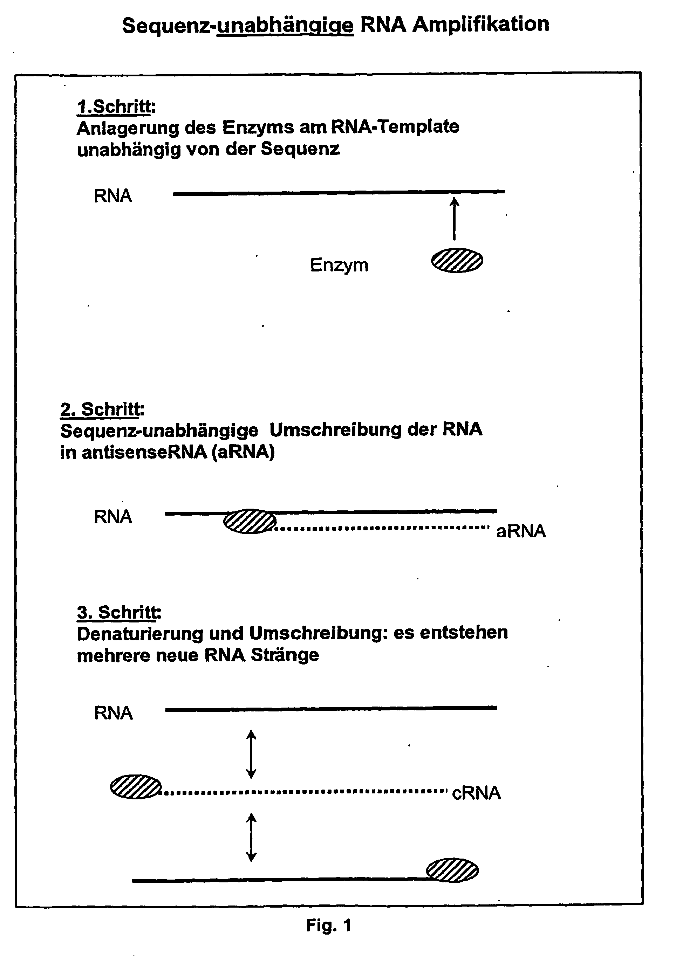

Im erfindungsgemäßen Verfahren erfolgt die Anlagerung der RNA-abhängigen RNA-Polymerase an die RNA-Matrize Primer-unabhängig. Der Ablauf des sequenzunabhängigen RNA-Amplifikation einer einzelsträngigen RNA-Matrize ohne Einsatz einer RNA-Primers ist in **Figur 1** schematisch dargestellt.

Bestandteil der Erfindung ist ein Verfahren zur Primer-unabhängigen Amplifikation von poly(C)-RNA, dadurch gekennzeichnet, dass kein Primer, der mit einem Abschnitt der RNA-Matrize hybridisiert, eingesetzt wird, dass GTP in erhöhter Konzentration, vorzugsweise 50 µM, verwendet wird und durch die RNA-abhänigige RNA-Polymerase entsprechend der Sequenz der RNA-Matrize verlängert wird.

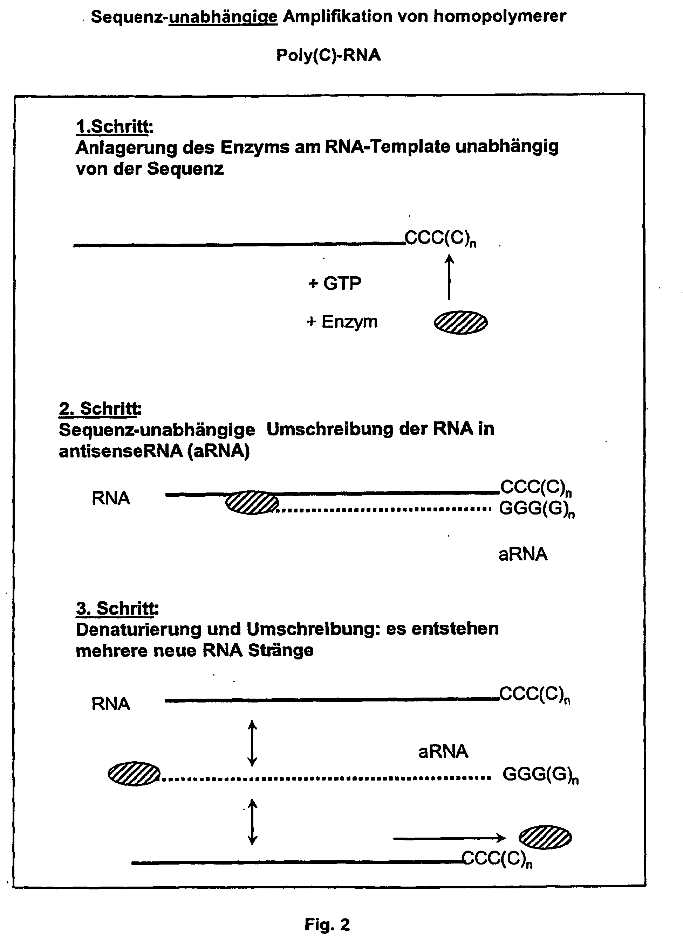

Der schematische Ablauf der sequenz-unabhängigen RNA-Synthese, ausgehend von einer poly(C)-RNA in Anwesenheit von 50 µM GTP, ist in Figur 2 dargestellt.

Die Anwendungsmöglichkeiten der erfindungsgemäßen Primer-unabhängigen Amplifikation von heteropolymerer oder poly(C)-RNA sind vielfältig. Eine Anwendung betrifft die unspezifische (RNA-Primer-unabhängige) Amplifikation von RNA, die ermöglichen kann, bisher nicht identifizierte Viren bzw. neue virale Varianten zu identifizieren.

Ein anderes Anwendungsgebiet der Erfindung ist die Herstellung von siRNA in vitro. Dies erfolgt bis dato mit Hilfe der T7-Polymerase. Es wird dafür
1. zwei komplementäre DNA-doppelstränge mittels PCR unter dem Einsatz eines T7-Promotor-Primers synthetisiert,
2. nach Umschreibung der DNA mit Hilfe der T7-Polymerase, werden beide resultierende komplementäre RNA-Stränge hybridisiert,
3. dann werden mittels RNAse I beide Stränge verdaut.

Diese Schritte dauern nach dem Stand der Technik in der Regel 24 bis 48 Stunden, und benötigen ebenso das notwendige "Know-How". Die Erfindung ermöglicht demgegenüber in 1,5 Stunden und ausgehend von einer RNA-Sequenz die direkte, effiziente und einfache Herstellung doppelsträngiger RNA, ohne die Notwendigkeit eines PCR-Schrittes, der *in vitro* Transkription und der Hybridisierung der RNA (die eventuell sub-optimal verlaufen kann).

Anhand der folgenden Figuren und Ausführungsbeispiele wird die Erfindung näher erläutert. Dabei zeigen
- **Figur 1:**: **Schematischer Ablauf der sequenz-unabhängigen RNA-Amplifikation**
- **Figur 2:**: **Schematischer Ablauf der sequenz-unabhängigen RNA-Synthese ausgehend von einer poly(C)-RNA in Anwesenheit von 50 µM GTP**

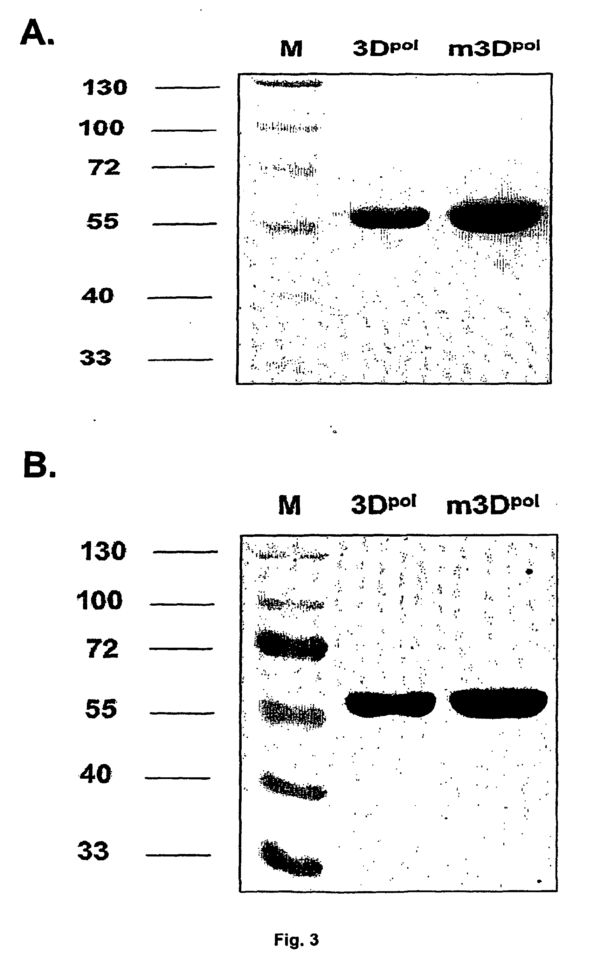

- **Figur 3:**: **Expression und Reinigung von Norovirus 3D^{pol} in *E. coli*.**
(A) SDS-PAGE-Analyse von in *E. coli* exprimierter, gereinigter, rekombinanter Norovirus 3D^{pol} mit einem C-terminalen His-tag. 3D^{pol}, Wildtyp- Norovirus 3D^{pol}. m3D^{pol}, Norovirus 3D^{pol} mit einer Mutation in der ,active site' (YGD_{343G}D_{344G}). M, Molekulargewichtsmarker (KDa).
(B) Western-Blot-Analyse von gereinigter rekombinanter Norovirus 3D^{pol} unter Verwendung eines Penta-Histidin-Antikörpers (monoklonaler Maus-Antikörper, Qiagen) zur Detektion von Proteinen mit einem C-terminalen His-tag. 3D^{pol}, Wildtyp-Norovirus 3D^{pol}. Norovirus 3D^{pol} mit einer Mutation in der ,active site' (YGD_{343G}D_{344G}). M, Molekulargewichtsmarker (KDa).

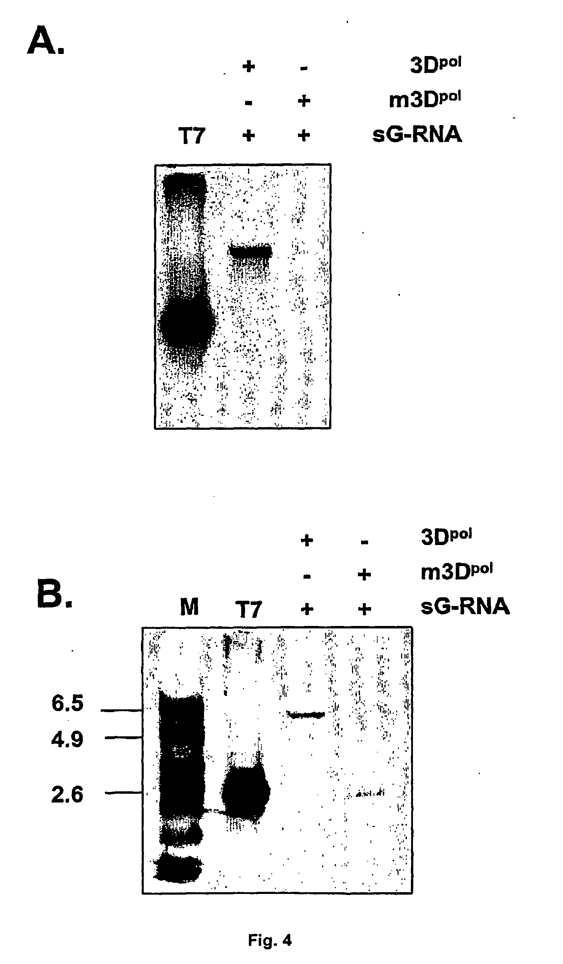

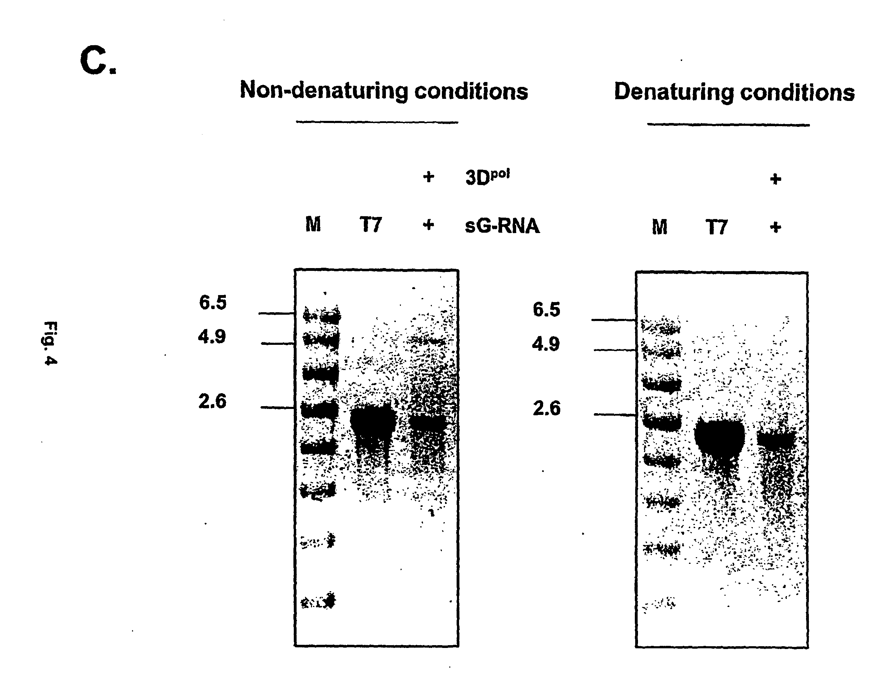

- **Figur 4:**: **RNA-Synthese mit Norovirus 3D^{pol}**
(A) Die RNA-Synthese wurde in Anwesenheit einer synthetischen subgenomischen RNA (sG-RNA) als Template sowie in Anwesenheit von Wildtyp-Norovirus 3D^{pol} oder einer Norovirus 3D^{pol} mit einer Mutation in der 'active site' (YGD_{343G}D_{344G}) wie angegeben untersucht. Die Reaktionsprodukte wurden auf einem nicht-denaturierenden Agarosegel analysiert und mittels Autoradiographie visualisiert. T7, synthetische subgenomische RNA, die durch in vitro Transkription mit T7 hergestellt wurde.
(B) Ethidiumbromid-Färbung desselben Gels und Visualisierung der Reaktionsprodukte durch UV-Transillumination. Die bei der Reaktion mit m3D^{pol} anstelle von Wildtyp 3D^{pol} sichtbare verbleibende Bande stammt von dem in der Reaktion eingesetzten synthetischen subgenomischen RNA- Template. M, RNA Molekulargewichtsmarker.
(C) Strangseparationsanalyse des Reaktionsprodukts der *in vitro* RNA-Synthese mit Norovirus 3D^{pol}. Das Reaktionsprodukt wurde durch RNA-Synthese mit Norovirus 3D^{pol} (3D^{pol} ) mit einer synthetischen subgenomischen RNA (sG- RNA) als Template hergestellt. Die Reaktionsprodukte wurden auf nicht- denaturierenden (0.5 M; links) und denaturierenden (1.25 M; rechts) Formaldehyde-Agarosegelen sichtbar gemacht. T7, synthetische subgenomische RNA, die mittels *in vitro* Transkription mit T7 hergestellt wurde. M, RNA Molekulargewichtsmarker.

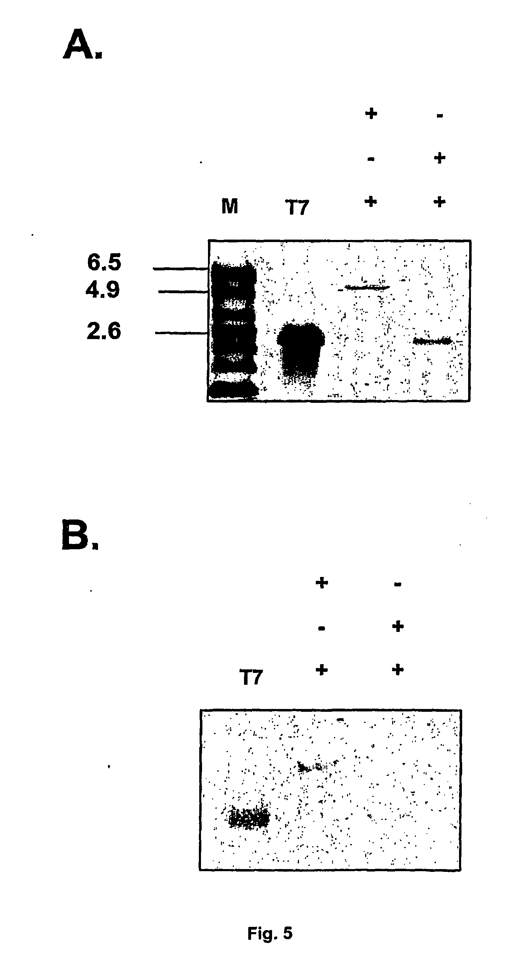

- **Figur 5:**: **Northern-Blot-Analyse der Norovirus 3D^{pol} Syntheseprodukte**
(A) Die RNA-Synthese wurde in Anwesenheit einer synthetischen subgenomischen RNA (sG-RNA) als Template sowie in Anwesenheit von Wildtyp-Norovirus 3D^{pol} oder einer Norovirus 3D^{pol} mit einer Mutation in der 'active site' (YGD_{343G}D_{344G}) wie angegeben durchgeführt. Die Reaktionsprodukte wurden auf einem nicht-denaturierenden Agarosegel analysiert und nach Ethidiumbromidfärbung mittels UV-Transillumination visualiert. T7, synthetische subgenomische RNA, die mittels *in vitro* Transkription mit T7 hergestellt wurde. M, RNA Molekulargewichtsmarker. Die bei der Reaktion mit m3D^{pol} anstelle von Wildtyp 3D^{pol} sichtbare verbleibende Bande stammt von dem in der Reaktion eingesetzten Template.
(B) Hybridisierung einer (-)-strängigen RNA-Probe mit den Syntheseprodukten der Norovirus 3D^{pol}. T7, synthetische subgenomische RNA, die mittels *in vitro* Transkription mit T7 hergestellt wurde. sG-RNA, als Template eingesetzte synthetische subgenomische RNA. 3D^{pol}, Wildtyp-Norovirus 3D^{pol}. Norovirus 3D^{pol} mit einer Mutation in der 'active site' (YGD_{343G}D_{344G}).

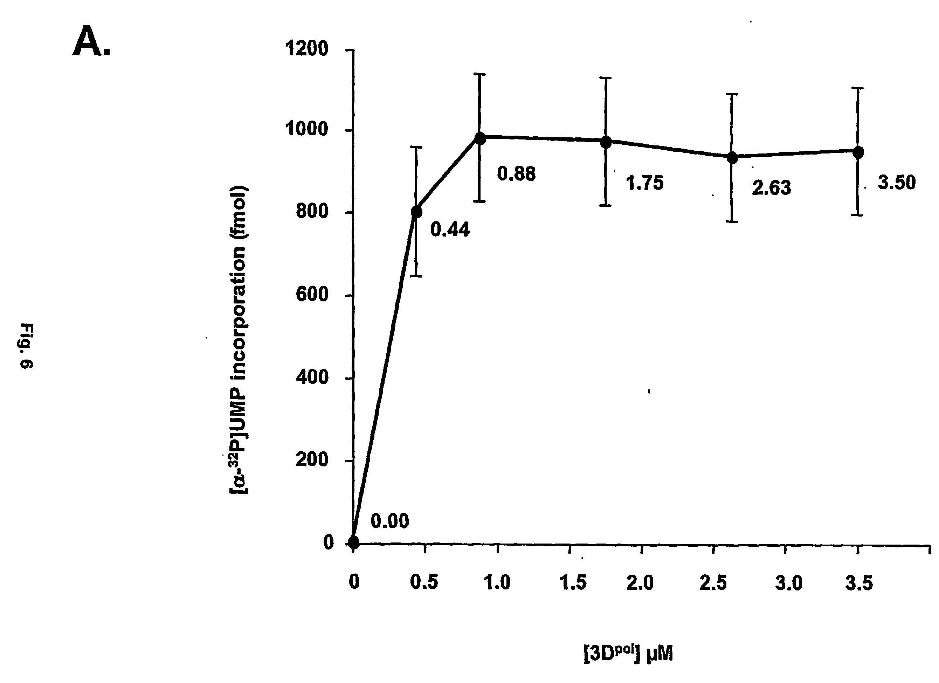

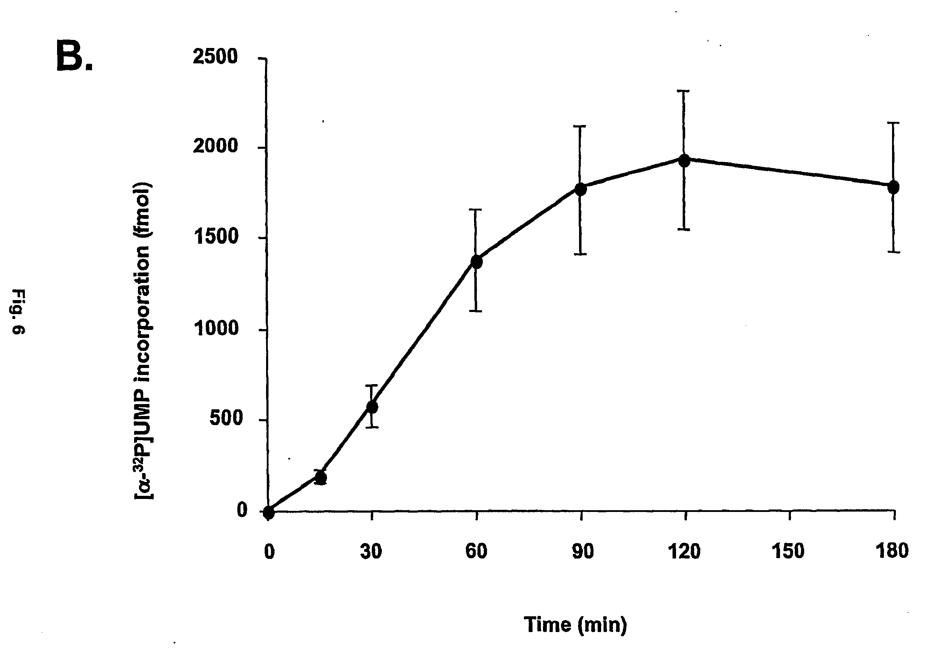

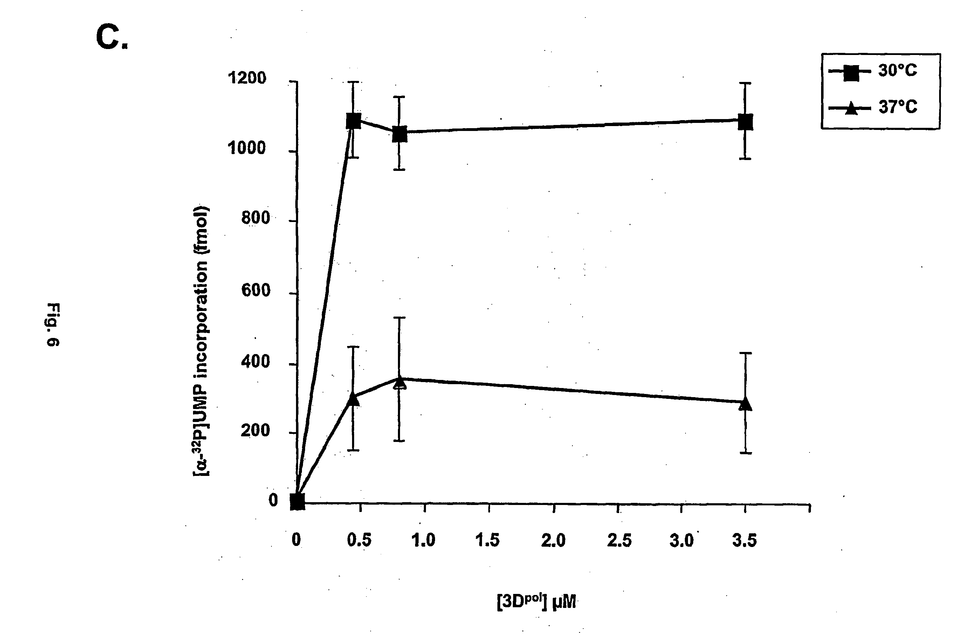

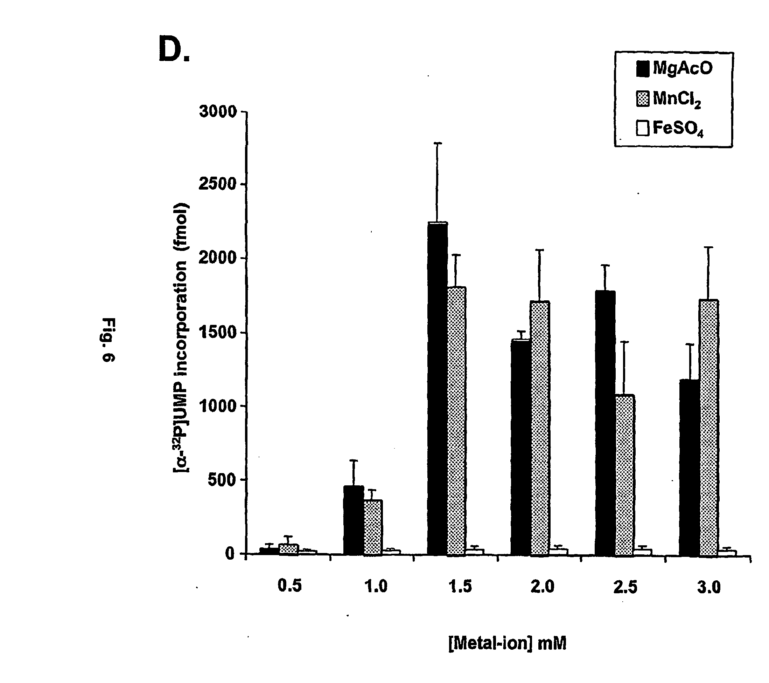

- **Figur 6:**: **Analyse der Konzentrationsabhängigkeit, der Temperaturabhängigkeit und des Zeitverlaufs der Norovirus 3D^{pol}-Aktivität.** Bei allen Reaktionen wurde subgenomische RNA als Template eingesetzt.
(A) Konzentrationsabhängige Aktivität der Norovirus 3D^{pol}. Norovirus 3D^{pol} Konzentration (µM) wie angegeben. Die Reaktion wurde für 2 h bei 30°C durchgeführt. Für jede Konzentration sind Mittelwert und Standardabweichung dreier unabhängiger Experimente gezeigt.
(B) Zeitverlaufsanalyse der Norovirus 3D^{pol}-Aktivität. Einbau von [α-³²P]UMP wie angegeben. Die Reaktion wurde bei 30°C durchgeführt und 3 µM Norovirus 3D^{pol} wurden eingesetzt. Für jeden Zeitpunkt sind Mittelwert und Standardabweichung dreier unabhängiger Experimente Experimenten gezeigt.
(C) Temperaturabhängige Aktivität der Norovirus 3D^{pol}. Einbau von [α-³²P]UMP wie angegeben. Die Reaktionszeit war 2 h. Für jede Konzentration von Norovirus 3D^{pol} sind Mittelwert und Standardabweichung dreier unabhängiger Experimente gezeigt.
(D) Metallionen-Abhängigkeit der Norovirus 3D^{pol}-Aktivität. Die RNA-Synthese wurde über 2 h bei 30°C in Gegenwart von 3 µM Norovirus 3D^{pol}, subgenomischer RNA als Template (0.024 µM) und steigender Konzentrationen (0.5 bis 3 mM) von MgAcO, MnCl₂ oder FeSO₄ durchgeführt. Einbau von [α-³²P]UMP wie angegeben. Für jede Konzentration sind Mittelwert und Standardabweichung dreier unabhängiger Experimente gezeigt.

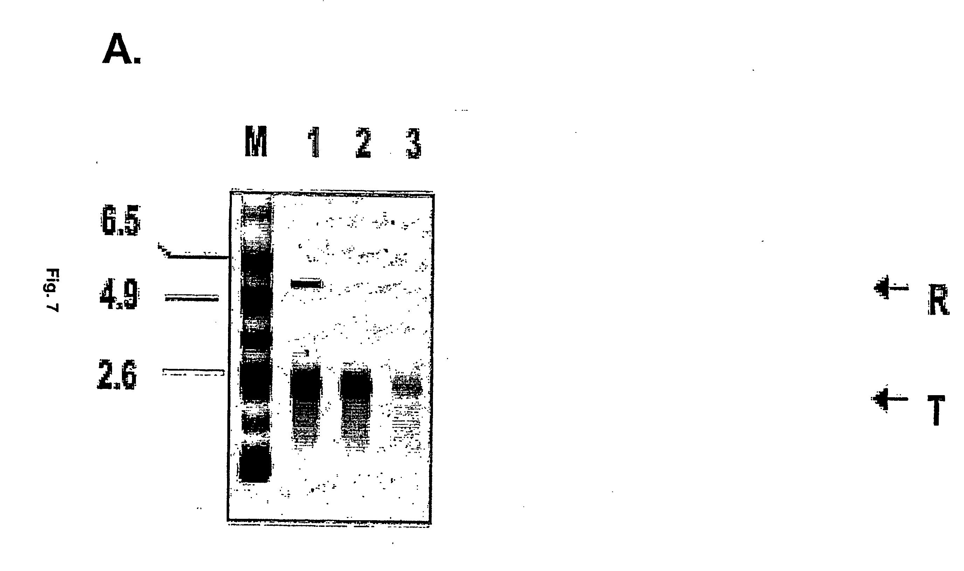

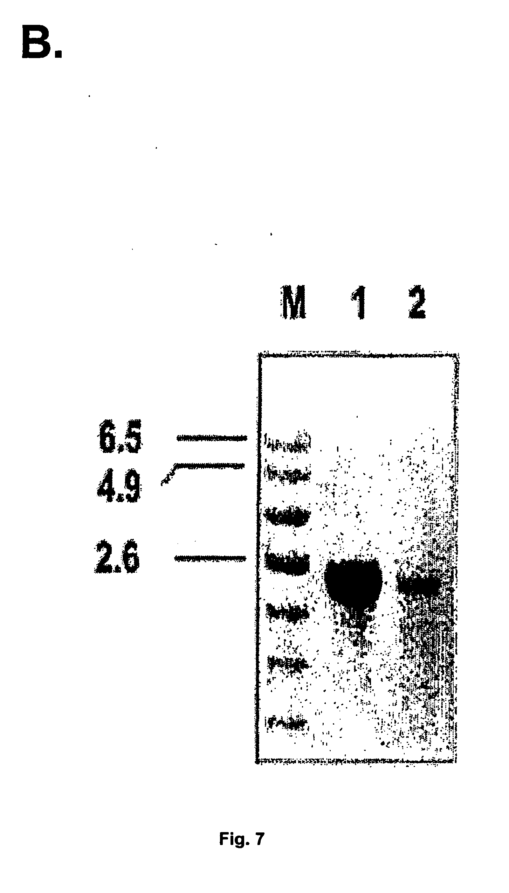

- **Figur 7:**: ***De novo* Initiation der RNA-Synthese an Norovirus antisubgenomischer RNA**
Bei allen Reaktionen wurde synthetische anti-subgenomische RNA als Template für die Replikationsreakton eingesetzt. Die RNA-Reaktionsprodukte wurden auf nativen Agarosegelen analysiert und nach Ethidiumbromidfärbung durch UV-Transillumination visualisiert.
(A) Bahnen 1 bis 3: RNA-Synthese in Gegenwart von Wildtyp 3D^{pol}, mutierter 3D^{pol} bzw. in Abwesenheit von 3D^{pol}. T, Template-RNA. R, Replikationsprodukt. M, RNA-Molekulargewichtsmarker (Kb).
(B) Strangseparationsanalyse der Replikationsprodukte. Die Reaktionsprodukte wurden auf Formaldehyd-Agarosegelen analysiert und nach Ethidiumbromidfärbung durch UV-Transillumination visualisiert. Bahnen 1 und 2, Template (anti-subgenomische RNA) bzw. Norovirus 3D^{pol} Replikationsprodukt. M, RNA Molekulargewichtsmarker (Kb).

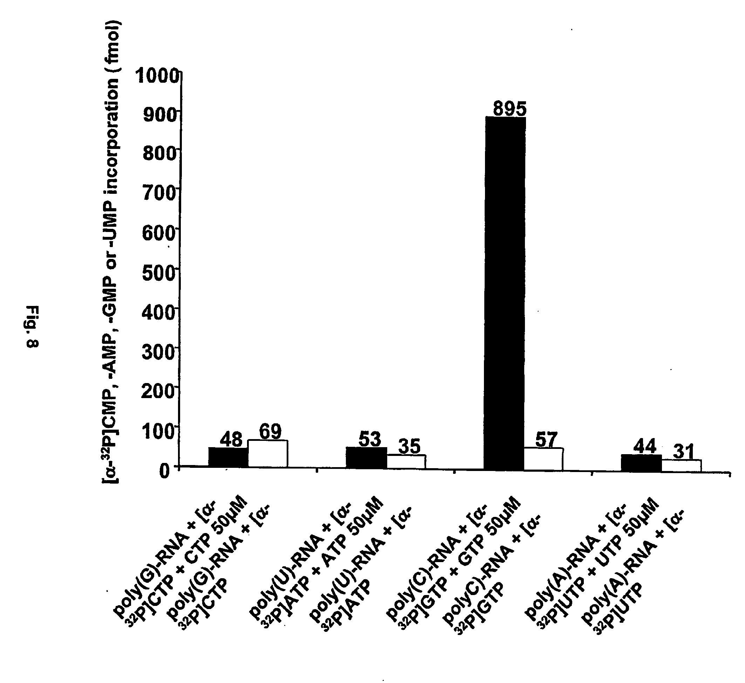

- **Figur 8:**: **Primer-unabhängige de *novo* Initiation der RNA Synthese an homopolymeren Templaten.** Die RNA-Synthese wurde in Anwesenheit (schwarze Balken) oder in Abwesenheit (graue Balken) von kaltem CTP, ATP, GTP bzw. UTP für poly(rG), poly(rU), poly(rC) bzw. poly(rA)-Templates durchgeführt. Der Einbau von [α-³²P]CMP, [α-³²P]AMP, [α-³²P]GMP, oder [α-³²P]UMP wurde nach TCA-Präzipitation und Sammeln von G/C-Glassfaserfiltern gemessen. Die Einbauwerte sind angegeben.

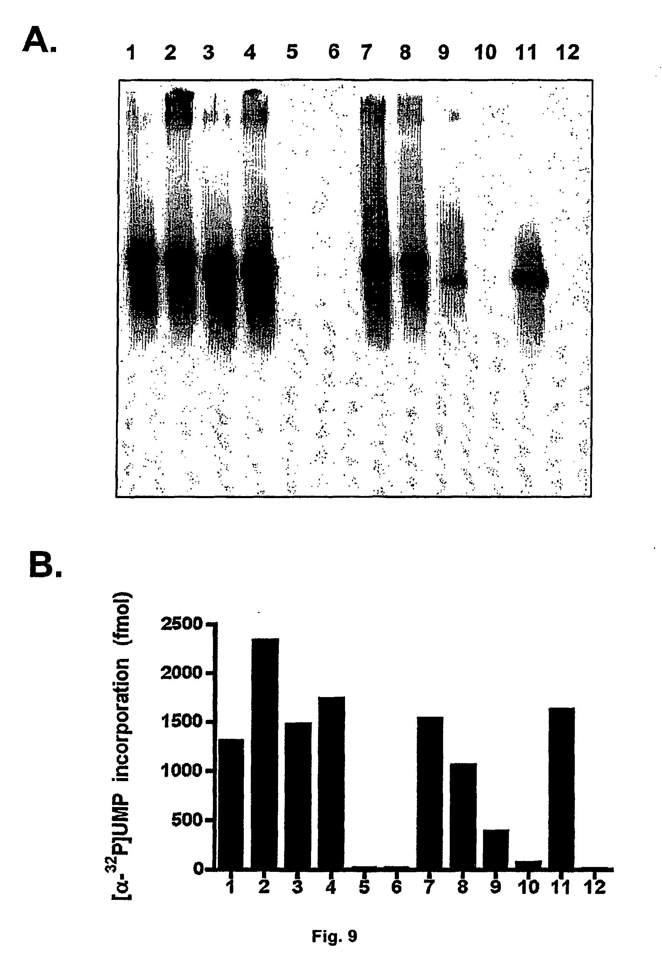

- **Figur 9:**: **Ampliflkation viraler und eukaryontischer RNA mit dem Norovirus RNA- Polymerase-Enzym.**
(A) Autoradiogramm der Inkorporation von [α-³²P]UMP. Reaktion 1, Subgenomische Norovirus RNA; Reaktion 2, Subgenomische Norovirus RNA mit einer 60 Nukleotid Deletion am 5'-Ende der RNA; Reaktion 3, Subgenomische Norovirus RNA mit einer 60 Nukleotid Deletion am 3'-Ende der RNA; Reaktion 4, Subgenomische Norovirus RNA mit einer 60 Nukleotid Deletion am 5'-Ende und 3'-Ende der RNA; Reaktion 5 u. 6, negative Kontrolle: Reaktion 7; Subgenomische Astrovirus RNA (Serotyp 1); Reaktion 8; Subgenomische Astrovirus RNA (Serotyp 2); Reaktion 9, eukaryontische RNA des elF4-Gens von *Xenopus Laevi,* Reaktion 10, Subgenomische Norovirus DNA: Reaktion 11; T7- transkriberte RNA des elF4-Gens von *Xenopus Laevi;* Reaktion 12, negative Kontrolle.
(B) Quantifizierung der Inkorporation von [α-³²P]UMP. Die Nummerierung der Reaktionen bezieht sich auf die Proben der **Figur 9 (A)****.**

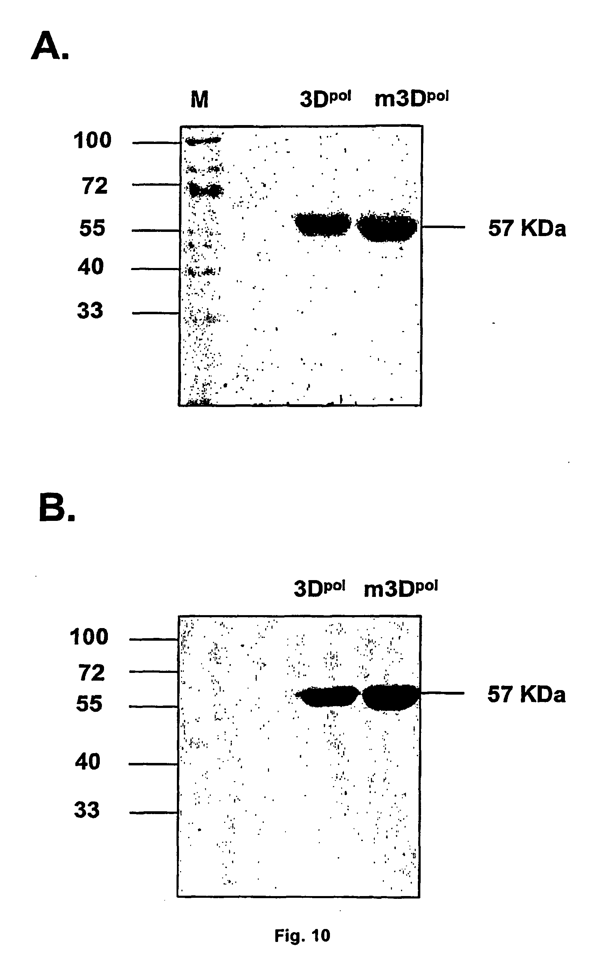

- **Figur 10:**: **Expression und Reinigung von Sapovirus 3D^{pol} in *E coli.***
(A) SDS-PAGE Analyse von gereinigter rekombinanter Sapovirus 3D^{pol} die in *E. coli* exprimiert wurde und einen C-terminalen His-tag trägt. 3D^{pol}, Wildtyp- Sapovirus 3D^{pl}. m3D^{pol}, Sapovirus 3D^{pol} mit einer Mutation in der 'active site' (YGD_{343G}D_{344G}). M, Molekulargewichtsmarker (KDa).
(B) Western-Blot Analyse von gereinigter rekombinanter Sapovirus 3D^{pol} unter Verwendung eines Penta-Histidin-Antikörpers (monoklonaler Maus-Antikörper, Qiagen) zur Detektion von Proteinen mit einem C-terminalen His-tag. 3D^{pol}, Wildtyp- Sapovirus 3D^{pol}. m3D^{pol}, Sapovirus 3D^{pol} mit einer Mutation in der 'active site' (YGD_{343G}D_{344G}), M, Molekulargewichtsmarker (KDa).

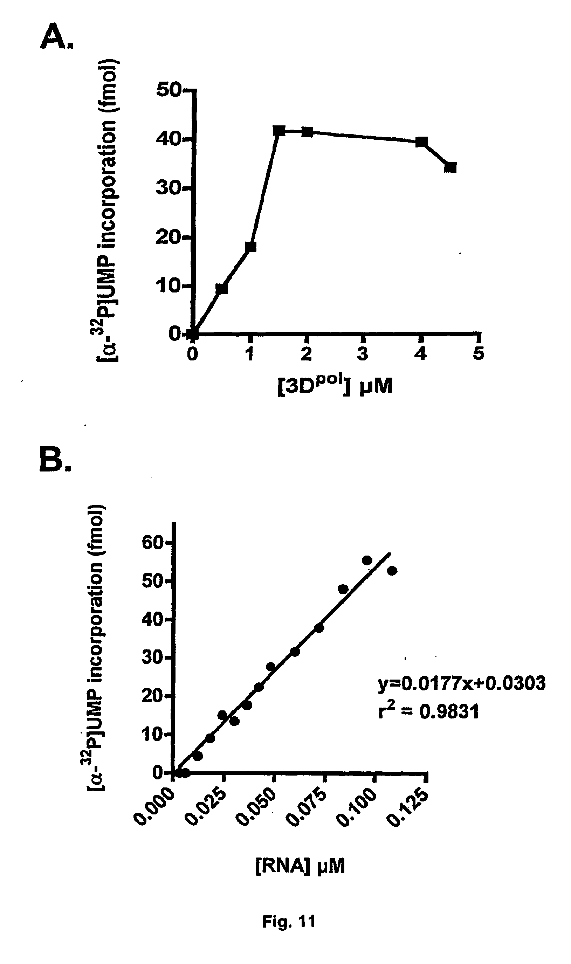

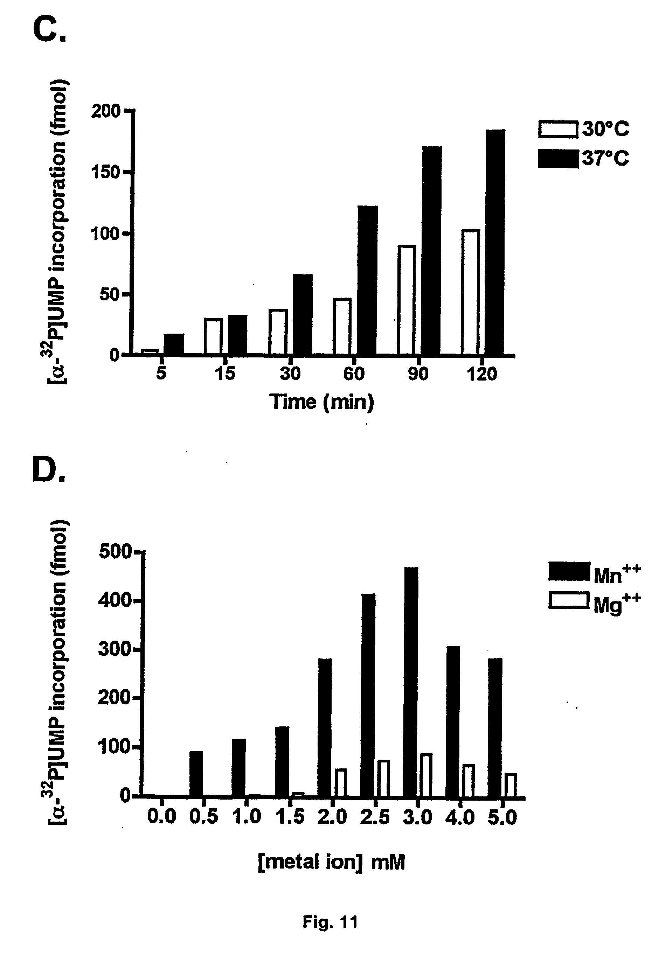

- **Figur 11:**: **Konzentrationsabhängigkeit, Substratabhängigkeit, Temperaturabhängigkeit und Metallionenabhänglgkeit der Sapovirus 3D^{pol} Aktivität.** Bei allen Reaktionen wurde subgenomische RNA als Template eingesetzt.
(A) Konzentrationsabhängige Aktivität der Sapovirus 3D^{pol}. Die RNA-Synthese wurde in Gegenwart von 0.024 µM RNA und ansteigenden Konzentrationen von Sapovirus 3D^{pol} (0.0 bis 5.0 µM) durchgeführt. Einbau von [α-³²P]UMP wie angegeben. Die Reaktion wurde 2 h bei 37°C durchgeführt.
(B) Substratabhängigkeit der Sapovirus 3D^{pol} Aktivität. Die RNA-Synthese wurde in Gegenwart von 3 µM Sapovirus 3D^{pol} und ansteigenden Konzentrationen von subgenomischer RNA als Template (0.00. bis 0.100 µM) für 2 h bei 37°C durchgeführt. Einbau von [α-³²P]UMP wie angegeben.
(C) Temperaturabhängige Activität von Sapovirus 3D^{pol}. Die Reaktion wurde bei 30°C oder 37°C durchgeführt und zu den angegebenen Zeiten gestoppt. Bei allen Reaktionen wurden 3 µM Sapovirus 3D^{pol} eingesetzt. Einbau von [α- ³²P]UMP wie angegeben.
(D) Metallionenabhängigkeit der Sapovirus 3D^{pol}-Aktivität. Die RNA-Synthese wurde in Gegenwart von 3 µM Sapovirus 3D^{pol} mit subgenomischer RNA als Template (0.024 µM) und steigenden Konzentrationen (0.5 bis 5 mM) von MgAcO oder MnCl₂ bei 37°C für 2 h durchgeführt. Einbau von [α-³²P]UMP wie angegeben.

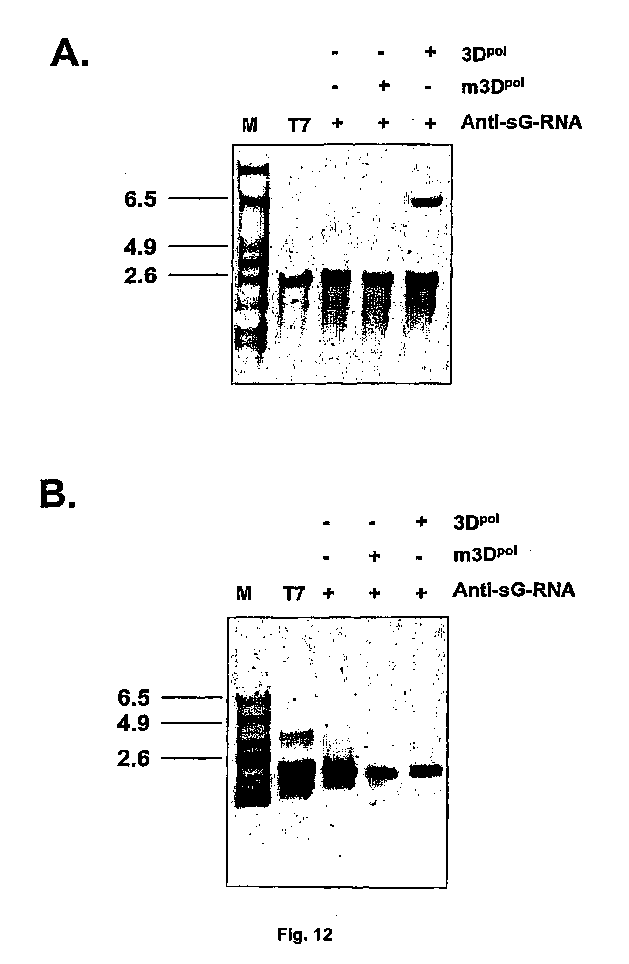

- **Figur 12:**: ***De novo* Initiation der RNA-Synthese an Sapovirus anti-subgenomischer RNA.**
(A) Die RNA-Synthese wurde in Gegenwart einer synthetischen anti- subgenomischen RNA (Anti-sG-RNA) als Template und Wildtyp-Sapovirus 3D^{pol} (3D^{pol}) oder einer in der 'active site' mutierten 3D^{pol} (m3D^{pol}, YGD_{343G}D_{344G}), wie angegeben. Die Reaktionsprodukte wurden auf nicht- denaturierenden Agarosegelen analysiert und nach Ethidiumbromidfärbung durch. UV Transillumination visualisiert. Die sichtbare verbleibende Bande bei der Reaktion, in der m3D^{pol} anstelle von Wildtyp 3D^{pol} eingesetzt wurde, resultiert aus dem in der Reaktion eingesetzten synthetischen anti- subgenomischen RNA-Template. T7, synthetische subgenomische RNA, die mittels *in vitro* Transkription mit T7 hergestellt wurde. M, RNA- Molekulargewichtsmarker.
(B) Strangseparationsanalyse des Reaktionsprodukts der in vitro RNA-Synthese mit Sapovirus 3D^{pol}. Das Reaktionsprodukt wurde wie angegeben durch RNA- Synthese mit Sapovirus 3D^{pol} unter Verwendung einer synthetischen anti- subgenomischen RNA (Anti-sG-RNA) als Template erhalten. Die Reaktionsprodukte wurden auf denaturierenden Formaldehyd-Agarosegelen visualisiert. T7, synthetische anti-subgenomische RNA, die mittels *in vitro* Transkription mit T7 hergestellt wurde. M, RNA-Molekulargewichtsmarker.

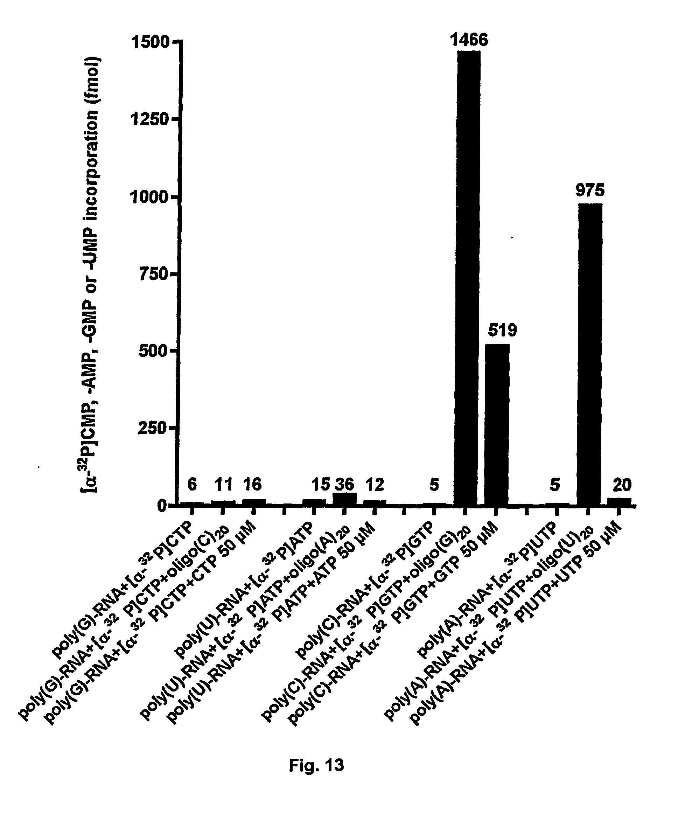

- **Figur 13:**: ***De novo* Initiation der RNA-Synthese durch Sapovirus 3D^{pol} an homopolymeren Templates.**
Die RNA-Synthese wurde durchgeführt, indem poly(G)-RNA, poly(U)-RNA, poly(C)- RNA und poly(A)-RNA Templates mit [α-³²P]CTP, [α-³²P]ATP, [α-³²P]GTP, bzw. [α- ³²P]UTP und mit oder ohne 3 µM eines Oligo(C)₂₀, Oligo(A)₂₀, Oligo (G)₂₀ bzw. Oligo(U)₂₀-RNA-Primers inkubiert wurden. Parallel dazu wurde die RNA-Synthese in Abwesenheit eines Oligo(RNA)-Primers, aber in Anwesenheit von 50 µM kaltem CTP, ATP, GTP bzw. UTP für poly(G)-RNA, poly(U)-RNA, poly(C)-RNA bzw. poly(A)-RNA Templates. Einbau von [α-³²P]CMP, [α-³²P]AMP, [α-³²P]GMP, oder [α-³²P]UMP as wie angegeben.

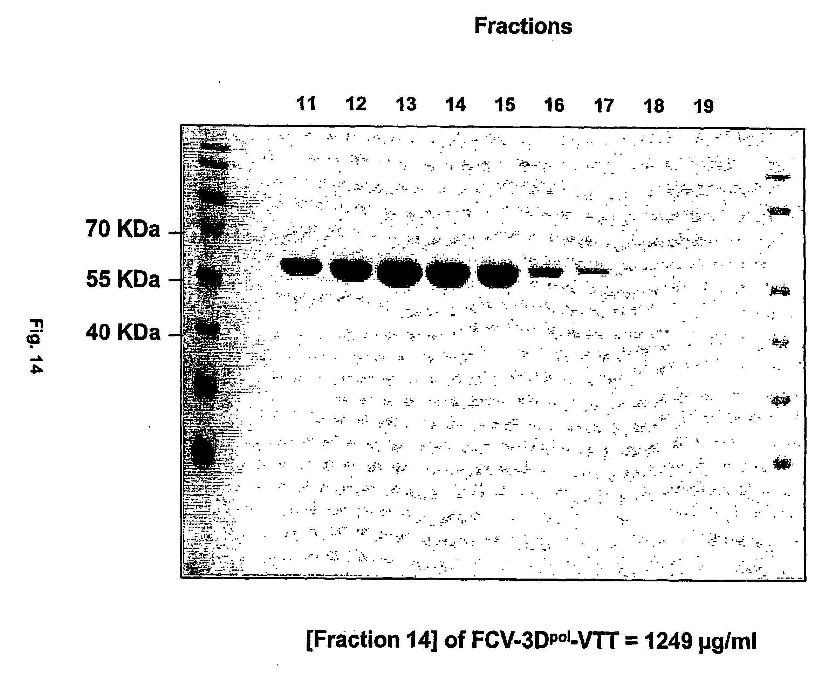

- **Figur 14:**: **Expression und Reinigung vom Vesivirus 3D^{pol} in *E*. *coli.***
(A) SDS-PAGE Analyse von in *E*. *coli* exprimierter, gereinigter, rekombinanter Vesivirus 3D^{pol} mit einem C-terminalen His-tag. Gezeigt sind die Fractionen 11 bis 19 nach Affinitätsreinigung an einer Ni-NTA-Säule sowie die Konzentration von Fraktion 14. M, Molekulargewichtsmarker (KDa).

### Ausführungsbeispiel 1

### Verfahren zur Herstellung rekombinanter Norovirus RdRP

Die cDNA der Norovirus-RdRP wurde durch PCR aus dem Norovirus-Klon pUS-Norll (Genbank accession number: AY741811) erhalten. Sie wurde in den pET-28b(+)-Vektor (Novagen) kloniert, der Expressionsvektor wurde sequenziert und in E.coli CL21 (DE3)pLysS transformiert. Die Zellen wurden bei 37°C in Luria-Bertani-Medium mit Kanamycin (50 mg/L) kultiviert. Bei einer optischen Dichte von 0.6 (OD600) wurde die Proteinexpression durch den Zusatz von Isopropyl-β-D-thiogalactopyranosid (IPTG) mit einer Endkonzentration von 1 mM induziert. Die Kulturen wurden dann bei 25 °C über Nacht inkubiert. Die aus 250 ml Kultur erhaltenen Zellpellets wurden einmal in 4 mi phosphate buffered saline (PBS) und 1 % Triton X 100 (Sigma) gewaschen. Die Zellen wurden bei 37 °C für 15 Minuten mit DNase (10 U/ml) behandelt, auf Eis sonifiziert und in 40 ml Bindungspuffer (20 mM Tris/HCl, pH 7.9, 500 mM NaCl, 5 mM Imidazol) resuspendiert. Nach der Zentrifugation bei 4300 rpm bei 4 °C für 40 Minuten wurde das geklärte Lysat erhalten. Die mit einem His6-tag versehene Norovirus-RdRP wurde an eine Ni-Nitrilotriacetessigsäure (NTA)-Sepharosematrix (Novagen) gebunden, die mit dem Bindungspuffer prääquilibriert worden war. Das gebundene Protein wurde mit dem Bindungspuffer, der 60 mM Imidazol enthielt, gewaschen und mit Bindungspuffer, der 1 M Imidazol enthielt, eluiert. Das eluierte Protein wurde gegen Puffer A dialysiert (25 mM Tris-hCl, pH 8.0, 1 mM ß-Mercaptoethanol, 100 mM NaCl, 5 mM MgCl2, 10 % Glycerin, 0.1 % Triton X). Die Proteinkonzentration wurde mit dem BCA proteinassay Kit (Pierce) auf der Grundlage der Biuret-Reaktion bestimmt. Das Enzym wurde in einem Endvolumen von 50 % Glycerin resuspendiert und bei -20°C aufbewahrt.

### Ausführungsbeispiel 2

### Sequenzunabhängige RNA-Amplifikation mit Norovirus-RdRP

Die Reaktionsmischung (50 µl) besteht aus 0.5 bis 1 µg RNA-Matrize, 10 µl des Reaktionspuffer (250 mM HEPES, pH 8.0, 15 mM Magnesiumacetat, 20 mM DTT), 50 U RNase-Inhibitor (RNAsin, Promega), je 0.4 mM von ATP, CTP, GTP, UTP, 3 µM der nach Ausführungsbeispiel 1 hergestellten Norovirus-RdRP. Die Reaktion wird bei 30°C für 2 Stunden durchgeführt. Die Reaktion wird durch die Zugabe von 50 µl der Stoplösung (4 M Ammoniumacetat, 100 mM EDTA) gestoppt. Die Reinigung erfolgt durch Phenol-Chloroform-Extraktion oder mit dem MEGAclear Kit (Ambion) nach den Herstelleranwelsungen. Die Transkriptionsprodukte werden nach Ethidiumbromid-Färbung durch UV-Transillumination auf TBE-gepufferten Agarosegelen sichtbar gemacht. Formamid-Agarosegele können ebenfalls verwendet werden.

### Ausführungsbeispiel 3

### Herstellung von siRNA mit Norovirus-RdRP

Die Reaktionsmischung (50 µl) besteht aus 0.5 bis 1 µg RNA-Matrize, 10 µl des Reaktionspuffer (250 mM HEPES, pH 8.0, 15 mM Magnesiumacetat, 20 mM DTT), 50 U RNase-inhibitor (RNAsin, Promega), je 0.4 mM von ATP, CTP, GTP, UTP, 3 µM der nach Ausführungsbeispfel 1 hergestellten Norovirus-RdRP. Die Reaktion wird bei 30 °C für 2 Stunden durchgeführt. Die Reaktion wird durch die Zugabe von 50 µl der Stoplösung (4 M Ammoniumacetat, 100 mM EDTA) gestoppt. Die Reinigung erfolgt durch Phenol-Chloroform-Extraktion oder mit dem MEGAclear Kit (Ambion) nach den Herstelleranwelsungen. Die Transkriptionsprodukte werden nach Ethidiumbromid-Färbung durch UV-Transillumination auf TBE-gepufferten 10 %igen Polyacrylamidgelen sichtbar gemacht. Formamid-Agarosegele können ebenfalls verwendet werden. Alternativ kann *armored-*RNA verwendet werden.

### Ausführungsbeispiel 4

### RNA-Synthese von poly(C)-poly(G) RNA-Hybrid mit Norovirus-RdRP

Die Reaktionsmischung (50 µl) besteht aus 0.5 bis 1 µg poly(C)-RNA-Matrize, oder eine RNA mit poly(C)-RNA am 3'-Ende, 10 µl des Reaktionspuffer (250 mM HEPES, pH 8.0, 15 mM Magnesiumacetat, 20 mM DTT), 50 U RNase-Inhibitor (RNAsin, Promega), 50 µM von GTP, und 3 µM der nach Ausführungsbeispiel 1 hergestellten Norovirus-RdRP. Die Reaktion wird bei 30 °C für 2 Stunden durchgeführt. Die Reaktion wird durch die Zugabe von 50 µl der Stoplösung (4 M Ammoniumacetat, 100 mM EDTA) gestoppt. Die Reinigung erfolgt durch Phenol-Chloroform-Extraktion oder mit dem MEGAclear Kit (Ambion) nach den Herstelleranweisungen. Die Transkriptionsprodukte werden nach Ethidiumbromid-Färbung durch UV-Transillumination auf TBE-gepufferten 10 %igen Polyacrylamidgelen sichtbar gemacht. Formamid-Agarosegele können ebenfalls verwendet werden. Alternativ kann *armored-*RNA verwendet werden.

## Patentansprüche

1. Verfahren zur Amplifikation von heteropolymerer RNA oder von poly(C)-RNA mit den Schritten
a. Anlagerung einer RNA-abhängigen RNA-Polymerase an die RNA-Matrize in Abwesenheit eines Oligonukleotid-Primers,
b. Umschreiben der RNA-Matrize in antisense-RNA durch die RNA-abhängige RNA-Polymerase,
c. Trennung des RNA/antisense-RNA-Duplexes in einzelne RNA-Stränge durch Hitzedenaturierung, chemische Denaturierung und/oder durch ein Enzym mit der Fähigkeit, doppelsträngige RNA in einzelsträngige RNA aufzutrennen,
d. ggf. Wiederholung der Schritte a. bis c.,
wobei die RNA-abhängige RNA-Polymerase eine "Rechte-Hand-Konformation" aufweist und die Aminosäuresequenz der RNA-abhängigen RNA-Polymerase die folgenden Sequenzabschnitte umfasst:
a. XXDYS
b. GXPSG
c. YGDD
d. XXYGL
e. XXXXFLXRXX
mit der Bedeutung
D: Aspartat
Y: Tyrosin
S: Serin
G: Glycin
P: Prolin
L: Leucin
F: Phenylalanin
R: Arginin
X: eine beliebige Aminosäure
und wobei die Umschreibung von poly(C)-RNA als Matrize in Anwesenheit von erhöhten GTP-Konzentrationen erfolgt.

2. Verfahren nach Anspruch 1, wobei die RNA-abhängige RNA-Polymerase eine RNA-abhängige RNA-Polymerase eines Virus aus der Familie der *Caliciviridae* ist.

3. Verfahren nach Anspruch 2, wobei die RNA-abhängige RNA-Polymerase eine RNA-abhängige RNA-Polymerase eines Norovirus, Sapovirus, Vesivirus oder Lagovirus ist.

4. Verfahren nach Anspruch 3, wobei die RNA-abhängige RNA-Polymerase ein Protein gemäß SEQ ID NO: 1, SEQ ID NO: 2, SEQ ID NO: 3, SEQ ID NO: 4, SEQ ID NO:5 oder SEQ ID NO: 6 ist.

5. Verfahren nach einem der Ansprüche 1 bis 4, wobei die enzymatische Trennung des RNA/antisense-RNA-Duplexes durch eine Helikase erfolgt.

6. Verfahren nach einem der Ansprüche 1 bis 6 zur Herstellung von siRNA *in vitro.*

7. Kit zur Amplifikation von heteropolymerer RNA oder von poly(C)-RNA in Abwesenheit eines Oligonukleotid-Primers, mindestens bestehend aus
a. einer RNA-abhängigen RNA-Polymerase gemäß der Definition in einem der Ansprüche 1 bis 4,
b. einem geeigneten Reaktionspuffer
c. NTPs,
d. einem Enzym mit der Fähigkeit, doppelsträngige in einzelsträngige RNA aufzutrennen, vorzugsweise einer Helikase,
e. ggf. RNAse-Inhibitor,
f. ggf. Stopplösung.

## Claims

1. A method for the amplification of heteropolymeric RNA or of poly(C) RNA comprising the steps of:
a. annealing of an RNA-dependent RNA polymerase to the RNA template in the absence of an oligonucleotide primer;
b. transcribing the RNA template into antisense RNA by the RNA-dependent RNA polymerase;
c. separating the RNA/antisense RNA duplex into individual RNA strands by heat denaturation, chemical denaturation and/or by an enzyme capable of separating double-stranded RNA into single-stranded RNA;
d. optionally, repeating steps a. to c;
wherein the RNA-dependent RNA polymerase has a "right hand conformation" and wherein the amino acid sequence of the RNA-dependent RNA polymerase comprises the following sequence motifs:
a. XXDYS
b. GXPSG
c. YGDD
d. XXYGL
e. XXXXFLXRXX
with the following meanings:
D: aspartate
Y: tyrosine
S: serine
G: glycine
P: proline
L: leucine
F: phenylalanine
R: arginine
X: any amino acid
and wherein transcribing poly(C) RNA as template occurs in the presence of elevated GTP concentrations.

2. The method of claim 1 wherein the RNA-dependent RNA polymerase is an RNA-dependent RNA polymerase of a virus of the *Caliciviridae* family*.*

3. The method of claim 2 wherein the RNA-dependent RNA polymerase is an RNA-dependent RNA polymerase of a norovirus, sapovirus, vesivirus or lagovirus.

4. The method of claim 3 wherein the RNA-dependent RNA polymerase is a protein according to SEO ID NO: 1, SEQ ID NO: 2, SEQ ID NO: 3, SEQ ID NO: 4, SEQ ID NO: 5 or SEQ ID NO: 6.

5. The method according to any one of claims 1 to 4 wherein the enzymatic separation of the RNA/antisense RNA duplex is carried out by a helicase.

6. The method according to any one of claims 1 to 5 for the production of siRNA *in vitro.*

7. A kit for the amplification of heteropolymeric RNA or of poly(C) RNA in the absence of an oligonucleotide primer, at least consisting of:
a. an RNA-dependent RNA polymerase as defined in any one of claims 1 to 4;
b. a suitable reaction buffer;
c. NTPs;
d. an enzyme capable of separating double-stranded RNA into single stranded RNA, preferably a helicase;
e. optionally, RNAse inhibitor;
f. optionally, stop solution.

## Revendications

1. Procédé pour l'amplification d'ARN hétéropolymérique ou d'ARN poly(C) comprenant les étapes de:
a. fixation d'une ARN-polymérase ARN-dépendante sur la matrice d'ARN en l'absence d'amorce oligonucléotidique;
b. transcription de la matrice d'ARN en ARN antisens par l'ARN-polymérase ARN-dépendante;
c. séparation du duplex ARN/ARN antisens en brins d'ARN simples par dénaturation à chaud, dénaturation chimique et/ou par une enzyme ayant la capacité de défaire de l'ADN double brin en ADN simple brin,
d. le cas échéant la reprise des étapes a à c,
dans lequel l'ARN-polymérase ARN-dépendante présente une "conformation main droite" et la séquence d'acides aminés de l'ARN-polymérase ARN-dépendante comprend les parties de séquence suivantes :
a. XXDYS
b. GXPSG
c. YGDD
d. XXYGL
e. XXXXFLXRXX
avec la signification:
D: aspartate
Y: tyrosine
S: sérine
G: glycine
P: proline
L: leucine
F: phénylalanine
R: arginine
X: n'importe quel acide aminé
et dans lequel la transcription de l'ARN poly(C) en tant que matrice a lieu en présence de concentrations en GTP élevées.

2. Procédé selon la revendication 1, dans lequel l'ARN-polymérase ARN-dépendante est une ARN-polymérase ARN-dépendante d'un virus de la famille des *Caliciviridae.*

3. Procédé selon la revendication 2, dans lequel l'ARN-polymérase ARN-dépendante est une ARN-polymérase ARN-dépendante d'un Norovirus, Sapovirus, Vesivirus ou Lagovirus.

4. Procédé selon la revendication 3, dans lequel l'ARN-polymérase ARN-dépendante est une protéine de séquence SEQ ID NO: 1, SEQ ID NO: 2, SEQ ID NO: 3, SEQ ID NO: 4, SEQ ID NO: 5 ou SEQ ID NO: 6.

5. Procédé selon l'une des revendications 1 à 4, dans lequel la séparation enzymatique du duplex ARN/ARN antisens est réalisée par une hélicase.

6. Procédé selon l'une des revendications 1 à 5 pour la production de siARN *in vitro.*

7. Kit pour l'amplification d'ARN hétéropolymérique ou d'ARN poly(C) en l'absence d'une amorce oligonucléotidique, consistant au moins en :
a. une ARN-polymérase ARN-dépendante telle que définie dans l'une des revendications 1 à 4,
b. un tampon de réaction approprié,
c. des NTPs;
d. une enzyme ayant la capacité de défaire de l'ARN double brin en simple brin, de préférence une hélicase,
e. le cas échéant, un inhibiteur de RNAse,
f. le cas échéant, une solution stop.


### SEQUENCE LISTING

<110> Technische universität Dresden
<120> RNA-abhängige RNA-Polymerase, verfahren und Kits zur Amplifikation und/oder Markierung von RNA
<130> T 0030 WOEPT2
<150> DE 10 2005 036 085.8
   <151> 2005-07-25
<150> DE 10 2006 008 219.2
   <151> 2006-02-13
<160> 6
<170> PatentIn version 3.3
<210> 1
   <211> 520
   <212> PRT
   <213> Norovirus
<400> 1
   Met Gly Gly Asp Ser Lys Gly Thr Tyr Cys Gly Ala Pro Ile Leu Gly
   1 5 10 15
   Pro Gly Ser Ala Pro Lys Leu Ser Thr Lys Thr Lys Phe Trp Arg Ser
   20 25 30
   Ser Thr Thr Pro Leu Pro Pro Gly Thr Tyr Glu Pro Ala Tyr Leu Gly
   35 40 45
   Gly Lys Asp Pro Arg Val Lys Gly Gly Pro Ser Leu Gln Gln Val Met
   50 55 60
   Arg Asp Gln Leu Lys Pro Phe Thr Glu Pro Arg Gly Lys Pro Pro Lys
   65 70 75 80
   Pro Ser Val Leu Glu Ala Ala Lys Lys Thr Ile Ile Asn Val Leu Glu
   85 90 95
   Gln Thr Ile Asp Pro Pro Glu Lys Trp Ser Phe Thr Gln Ala Cys Ala
   100 105 110
   Ser Leu Asp Lys Thr Thr Ser Ser Gly His Pro His His Met Arg Lys
   115 120 125
   Asn Asp Cys Trp Asn Gly Glu Ser Phe Thr Gly Lys Leu Ala Asp Gln
   130 135 140
   Ala Ser Lys Ala Asn Leu Met Phe Glu Gly Gly Lys Asn Met Thr Pro
   145 150 155 160
   Val Tyr Thr Gly Ala Leu Lys Asp Glu Leu Val Lys Thr Asp Lys Ile
   165 170 175
   Tyr Gly Lys Ile Lys Lys Arg Leu Leu Trp Gly Ser Asp Leu Ala Thr
               180                 185                 190
   Met Ile Arg Cys Ala Arg Ala Phe Gly Gly Leu Met Asp Glu Leu Lys
           195                 200                 205
   Ala His Cys Val Thr Leu Pro Ile Arg Val Gly Met Asn Met Asn Glu
       210                 215                 220
   Asp Gly Pro Ile Ile Phe Glu Arg His Ser Arg Tyr Lys Tyr His Tyr
   225                 230                 235                 240
   Asp Ala Asp Tyr Ser Arg Trp Asp Ser Thr Gln Gln Arg Ala Val Leu
                   245                 250                 255
   Ala Ala Ala Leu Glu Ile Met Val Lys Phe Ser Ser Glu Pro His Leu
               260                 265                 270
   Ala Gln Val Val Ala Glu Asp Leu Leu Ser Pro Ser Val Val Asp Val
           275                 280                 285
   Gly Asp Phe Lys Ile Ser Ile Asn Glu Gly Leu Pro Ser Gly Val Pro
       290                 295                 300
   Cys Thr Ser Gln Trp Asn Ser Ile Ala His Trp Leu Leu Thr Leu Cys
   305                 310                 315                 320
   Ala Leu Ser Glu Val Thr Asn Leu Ser Pro Asp Ile Ile Gln Ala Asn
                   325                 330                 335
   Ser Leu Phe Ser Phe Tyr Gly Asp Asp Glu Ile Val Ser Thr Asp Ile
               340                 345                 350
   Lys Leu Asp Pro Glu Lys Leu Thr Ala Lys Leu Lys Glu Tyr Gly Leu
           355                 360                 365
   Lys Pro Thr Arg Pro Asp Lys Thr Glu Gly Pro Leu Val Ile Ser Glu
       370                 375                 380
   Asp Leu Asn Gly Leu Thr Phe Leu Arg Arg Thr Val Thr Arg Asp Pro
   385                 390                 395                 400
   Ala Gly Trp Phe Gly Lys Leu Glu Gln Ser Ser Ile Leu Arg Gln Met
                   405                 410                 415
   Tyr Trp Thr Arg Gly Pro Asn His Glu Asp Pro Ser Glu Thr Met Ile
               420                 425                 430
   Pro His Ser Gln Arg Pro Ile Gln Leu Met Ser Leu Leu Gly Glu Ala
           435                 440                 445
   Ala Leu His Gly Pro Ala Phe Tyr Ser Lys Ile Ser Lys Leu Val Ile
   450 455 460
   Ala Glu Leu Lys Glu Gly Gly Met Asp Phe Tyr Val Pro Arg Gln Glu
   465 470 475 480
   Pro Met Phe Arg Trp Met Arg Phe Ser Asp Leu Ser Thr Trp Glu Gly
   485 490 495
   Asp Arg Asn Leu Ala Pro Ser Phe Val Asn Glu Asp Gly Val Glu Val
   500 505 510
   Asp Lys Leu Ala Ala Ala Leu Glu
   515 520
<210> 2
   <211> 517
   <212> PRT
   <213> Sapovirus
<400> 2
   Met Lys Asp Glu Phe Gln Trp Lys Gly Leu Pro Val Val Lys Ser Gly
   1               5                   10                  15
   Leu Asp Val Gly Gly Met Pro Thr Gly Thr Arg Tyr His Arg Ser Pro
               20                  25                  30
   Ala Trp Pro Glu Glu Gln Pro Gly Glu Thr His Ala Pro Ala Pro Phe
           35                  40                  45
   Gly Ala Gly Asp Lys Arg Tyr Thr Phe Ser Gln Thr Glu Met Leu Val
       50                  55                  60
   Asn Gly Leu Lys Pro Tyr Thr Glu Pro Thr Ala Gly Val Pro Pro Gln
   65                  70                  75                  80
   Leu Leu Ser Arg Ala Val Thr His Val Arg Ser Tyr Ile Glu Thr Ile
                   85                  90                  95
   Ile Gly Thr His Arg Ser Pro Val Leu Thr Tyr His Gln Ala Cys Glu
               100                 105                 110
   Leu Leu Glu Arg Thr Thr Ser Cys Gly Pro Phe Val Gln Gly Leu Lys
           115                 120                 125
   Gly Asp Tyr Trp Asp Glu Glu Gln Gln Gln Tyr Thr Gly Val Leu Ala
       130                 135                 140
   Asn His Leu Glu Gln Ala Trp Asp Lys Ala Asn Lys Gly Ile Ala Pro
   145                 150                 155                 160
   Arg Asn Ala Tyr Lys Leu Ala Leu Lys Asp Glu Leu Arg Pro Ile Glu
                   165                 170                 175
   Lys Asn Lys Ala Gly Lys Arg Arg Leu Leu Trp Gly Cys Asp Ala Ala
               180                 185                 190
   Thr Thr Leu Ile Ala Thr Ala Ala Phe Lys Ala Val Ala Thr Arg Leu
           195                 200                 205
   Gln Val Val Thr Pro Met Thr Pro Val Ala Val Gly Ile Asn Met Asp
       210                 215                 220
   Ser Val Gln Met Gln Val Met Asn Asp Ser Leu Lys Gly Gly Val Leu
   225                 230                 235                 240
   Tyr Cys Leu Asp Tyr Ser Lys Trp Asp Ser Thr Gln Asn Pro Ala Val
                   245                 250                 255
   Thr Ala Ala Ser Leu Ala Ile Leu Glu Arg Phe Ala Glu Pro His Pro
               260                 265                 270
   Ile Val Ser Cys Ala Ile Glu Ala Leu Ser Ser Pro Ala Glu Gly Tyr
           275                 280                 285
   Val Asn Asp Ile Lys Phe Val Thr Arg Gly Gly Leu Pro Ser Gly Met
       290                 295                 300
   Pro Phe Thr Ser Val Val Asn Ser Ile Asn His Met Ile Tyr Val Ala
   305                 310                 315                 320
   Ala Ala Ile Leu Gln Ala Tyr Glu Ser His Asn Val Pro Tyr Thr Gly
                   325                 330                 335
   Asn Val Phe Gln Val Glu Thr Val His Thr Tyr Gly Asp Asp Cys Met
               340                 345                 350
   Tyr Ser Val Cys Pro Ala Thr Ala Ser Ile Phe His Ala Val Leu Ala
           355                 360                 365
   Asn Leu Thr Ser Tyr Gly Leu Lys Pro Thr Ala Ala Asp Lys Ser Asp
       370                 375                 380
   Ala Ile Lys Pro Thr Asn Thr Pro Val Phe Leu Lys Arg Thr Phe Thr
   385                 390                 395                 400
   Gln Thr Pro His Gly Val Arg Ala Leu Leu Asp Ile Thr Ser Ile Thr
                   405                 410                 415
   Arg Gln Phe Tyr Trp Leu Lys Ala Asn Arg Thr Ser Asp Pro Ser Ser
               420                 425                 430
   Pro Pro Ala Phe Asp Arg Gln Ala Arg Ser Ala Gln Leu Glu Asn Ala
   435 440 445
   Leu Ala Tyr Ala Ser Gln His Gly Pro Val Val Phe Asp Thr Val Arg
   450 455 460
   Gln Ile Ala Ile Lys Thr Ala Gln Gly Glu Gly Leu Val Leu Val Asn
   465 470 475 480
   Thr Asn Tyr Asp Gln Ala Leu Ala Thr Tyr Asn Ala Trp Phe Ile Gly
   485 490 495
   Gly Thr Val Pro Asp Pro Val Gly His Thr Glu Gly Thr His Lys Ile
   500 505 510
   Val Phe Glu Met Glu
   515
<210> 3
   <211> 533
   <212> PRT
   <213> Vesivirus
<400> 3
   Met Lys Val Thr Thr Gln Lys Tyr Asp Val Thr Lys Pro Asp Ile Ser
   1 5 10 15
   Tyr Lys Gly Leu Ile Cys Lys Gln Leu Asp Glu Ile Arg Val Ile Pro
   20 25 30
   Lys Gly Thr Arg Leu His Val Ser Pro Ala His Thr Asp Asp Tyr Asp
   35 40 45
   Glu Cys Ser His Gln Pro Ala Ser Leu Gly Ser Gly Asp Pro Arg Cys
   50 55 60
   Pro Lys Ser Leu Thr Ala Ile Val Val Asp Ser Leu Lys Pro Tyr Cys
   65 70 75 80
   Glu Lys Thr Asp Gly Pro Pro His Asp Ile Leu His Arg Val Gln Arg
   85 90 95
   Met Leu Ile Asp His Leu Ser Gly Phe Val Pro Met Asn Ile Ser Ser
   100 105 110
   Glu Pro Ser Met Leu Ala Ala Phe His Lys Leu Asn His Asp Thr Ser
   115 120 125
   Cys Gly Pro Tyr Leu Gly Gly Arg Lys Lys Asp His Met Ile Gly Gly
   130 135 140
   Glu Pro Asp Lys Pro Leu Leu Asp Leu Leu Ser Ser Lys Trp Lys Leu
   145                 150                 155                 160
   Ala Thr Gln Gly Ile Gly Leu Pro His Glu Tyr Thr Ile Gly Leu Lys
                   165                 170                 175
   Asp Glu Leu Arg Pro Val Glu Lys Val Gln Glu Gly Lys Arg Arg Met
               180                 185                 190
   Ile Trp Gly Cys Asp Val Gly Val Ala Thr Val Cys Ala Ala Ala Phe
           195                 200                 205
   Lys Gly Val Ser Asp Ala Ile Thr Ala Asn His Gln Tyr Gly Pro Val
       210                 215                 220
   Gln Val Gly Ile Asn Met Asp Gly Pro Ser Val Glu Ala Leu Tyr Gln
   225                 230                 235                 240
   Arg Ile Arg Ser Ala Ala Lys Val Phe Ala Val Asp Tyr Ser Lys Trp
                   245                 250                 255
   Asp Ser Thr Gln Ser Pro Arg Val Ser Ala Ala Ser Ile Asp Ile Leu
               260                 265                 270
   Arg Tyr Phe Ser Asp Arg Ser Pro Ile Val Asp Ser Ala Ala Asn Thr
           275                 280                 285
   Leu Lys Ser Pro Pro Ile Ala Ile Phe Asn Gly Val Ala Val Lys Val
       290                 295                 300
   Thr Ser Gly Leu Pro Ser Gly Met Pro Leu Thr Ser Val Ile Asn Ser
   305                 310                 315                 320
   Leu Asn His Cys Leu Tyr Val Gly Cys Ala Ile Leu Gln Ser Leu Glu
                   325                 330                 335
   Ser Arg Asn Ile Pro Val Thr Trp Asn Leu Phe Ser Thr Phe Asp Met
               340                 345                 350
   Met Thr Tyr Gly Asp Asp Gly Val Tyr Met Phe Pro Met Met Phe Ala
           355                 360                 365
   Ser Val Ser Asp Gln Ile Phe Ala Asn Leu Thr Ala Tyr Gly Leu Lys
       370                 375                 380
   Pro Thr Arg Val Asp Lys Ser Val Gly Ala Ile Glu Pro Ile Asp Pro
   385                 390                 395                 400
   Glu Ser Val Val Phe Leu Lys Arg Thr Ile Thr Arg Thr Pro His Gly
                   405                 410                 415
   Ile Arg Gly Leu Leu Asp Arg Gly Ser Ile Ile Arg Gln Phe Tyr Tyr
   420 425 430
   Ile Lys Gly Glu Asn Ser Asp Asp Trp Lys Thr Pro Pro Lys Thr Ile
   435 440 445
   Asp Pro Thr Ser Arg Gly Gln Gln Leu Trp Asn Ala Cys Leu Tyr Ala
   450 455 460
   Ser Gln His Gly Pro Glu Phe Tyr Asn Lys Val Tyr Arg Leu Ala Glu
   465 470 475 480
   Lys Ala Val Glu Tyr Glu Glu Leu His Phe Glu Pro Pro Ser Tyr His
   485 490 495
   Ser Ala Leu Glu His Tyr Asn Asn Gln Phe Asn Gly Val Asp Thr Arg
   500 505 510
   Ser Asp Gln Ile Asp Ala Ser Val Met Thr Asp Leu His Cys Asp Val
   515 520 525
   Phe Glu Val Leu Glu
   530
<210> 4
   <211> 526
   <212> PRT
   <213> Artificial
<220>
   <223> Norovirus-RdRP mit einem Hisitiden-Marker
<400> 4
   Met Gly Gly Asp Ser Lys Gly Thr Tyr Cys Gly Ala Pro Ile Leu Gly
   1               5                   10                  15
   Pro Gly Ser Ala Pro Lys Leu Ser Thr Lys Thr Lys Phe Trp Arg Ser
               20                  25                  30
   Ser Thr Thr Pro Leu Pro Pro Gly Thr Tyr Glu Pro Ala Tyr Leu Gly
           35                  40                  45
   Gly Lys Asp Pro Arg Val Lys Gly Gly Pro Ser Leu Gln Gln Val Met
       50                  55                  60
   Arg Asp Gln Leu Lys Pro Phe Thr Glu Pro Arg Gly Lys Pro Pro Lys
   65                  70                  75                  80
   Pro Ser Val Leu Glu Ala Ala Lys Lys Thr Ile Ile Asn Val Leu Glu
                   85                  90                  95
   Gln Thr Ile Asp Pro Pro Glu Lys Trp Ser Phe Thr Gln Ala Cys Ala
               100                 105                 110
   Ser Leu Asp Lys Thr Thr Ser Ser Gly His Pro His His Met Arg Lys
           115                 120                 125
   Asn Asp Cys Trp Asn Gly Glu Ser Phe Thr Gly Lys Leu Ala Asp Gln
       130                 135                 140
   Ala Ser Lys Ala Asn Leu Met Phe Glu Gly Gly Lys Asn Met Thr Pro
   145                 150                 155                 160
   Val Tyr Thr Gly Ala Leu Lys Asp Glu Leu Val Lys Thr Asp Lys Ile
                   165                 170                 175
   Tyr Gly Lys Ile Lys Lys Arg Leu Leu Trp Gly Ser Asp Leu Ala Thr
               180                 185                 190
   Met Ile Arg Cys Ala Arg Ala Phe Gly Gly Leu Met Asp Glu Leu Lys
           195                 200                 205
   Ala His Cys Val Thr Leu Pro Ile Arg Val Gly Met Asn Met Asn Glu
       210                 215                 220
   Asp Gly Pro Ile Ile Phe Glu Arg His Ser Arg Tyr Lys Tyr His Tyr
   225                 230                 235                 240
   Asp Ala Asp Tyr Ser Arg Trp Asp Ser Thr Gln Gln Arg Ala Val Leu
                   245                 250                 255
   Ala Ala Ala Leu Glu Ile Met Val Lys Phe Ser Ser Glu Pro His Leu
               260                 265                 270
   Ala Gln Val Val Ala Glu Asp Leu Leu Ser Pro Ser Val Val Asp Val
           275                 280                 285
   Gly Asp Phe Lys Ile Ser Ile Asn Glu Gly Leu Pro Ser Gly Val Pro
       290                 295                 300
   Cys Thr Ser Gln Trp Asn Ser Ile Ala His Trp Leu Leu Thr Leu Cys
   305                 310                 315                 320
   Ala Leu Ser Glu Val Thr Asn Leu Ser Pro Asp Ile Ile Gln Ala Asn
                   325                 330                 335
   Ser Leu Phe Ser Phe Tyr Gly Asp Asp Glu Ile Val Ser Thr Asp Ile
               340                 345                 350
   Lys Leu Asp Pro Glu Lys Leu Thr Ala Lys Leu Lys Glu Tyr Gly Leu
           355                 360                 365
   Lys Pro Thr Arg Pro Asp Lys Thr Glu Gly Pro Leu Val Ile Ser Glu
       370                 375                 380
   Asp Leu Asn Gly Leu Thr Phe Leu Arg Arg Thr Val Thr Arg Asp Pro
   385 390 395 400
   Ala Gly Trp Phe Gly Lys Leu Glu Gln Ser Ser Ile Leu Arg Gln Met
   405 410 415
   Tyr Trp Thr Arg Gly Pro Asn His Glu Asp Pro Ser Glu Thr Met Ile
   420 425 430
   Pro His Ser Gln Arg Pro Ile Gln Leu Met Ser Leu Leu Gly Glu Ala
   435 440 445
   Ala Leu His Gly Pro Ala Phe Tyr Ser Lys Ile Ser Lys Leu Val Ile
   450 455 460
   Ala Glu Leu Lys Glu Gly Gly Met Asp Phe Tyr Val Pro Arg Gln Glu
   465 470 475 480
   Pro Met Phe Arg Trp Met Arg Phe Ser Asp Leu Ser Thr Trp Glu Gly
   485 490 495
   Asp Arg Asn Leu Ala Pro Ser Phe Val Asn Glu Asp Gly Val Glu Val
   500 505 510
   Asp Lys Leu Ala Ala Ala Leu Glu His His His His His His
   515 520 525
<210> 5
   <211> 523
   <212> PRT
   <213> Artificial
<220>
   <223> Sapovirus-RdRP mit einem Histidin-Marker
<400> 5
   Met Lys Asp Glu Phe Gln Trp Lys Gly Leu Pro Val Val Lys Ser Gly
   1               5                   10                  15
   Leu Asp Val Gly Gly Met Pro Thr Gly Thr Arg Tyr His Arg Ser Pro
               20                  25                  30
   Ala Trp Pro Glu Glu Gln Pro Gly Glu Thr His Ala Pro Ala Pro Phe
           35                  40                  45
   Gly Ala Gly Asp Lys Arg Tyr Thr Phe Ser Gln Thr Glu Met Leu Val
       50                  55                  60
   Asn Gly Leu Lys Pro Tyr Thr Glu Pro Thr Ala Gly Val Pro Pro Gln
   65                  70                  75                  80
   Leu Leu Ser Arg Ala Val Thr His Val Arg Ser Tyr Ile Glu Thr Ile
                   85                  90                  95
   Ile Gly Thr His Arg Ser Pro Val Leu Thr Tyr His Gln Ala Cys Glu
               100                 105                 110
   Leu Leu Glu Arg Thr Thr Ser Cys Gly Pro Phe Val Gln Gly Leu Lys
           115                 120                 125
   Gly Asp Tyr Trp Asp Glu Glu Gln Gln Gln Tyr Thr Gly Val Leu Ala
       130                 135                 140
   Asn His Leu Glu Gln Ala Trp Asp Lys Ala Asn Lys Gly Ile Ala Pro
   145                 150                 155                 160
   Arg Asn Ala Tyr Lys Leu Ala Leu Lys Asp Glu Leu Arg Pro Ile Glu
                   165                 170                 175
   Lys Asn Lys Ala Gly Lys Arg Arg Leu Leu Trp Gly Cys Asp Ala Ala
               180                 185                 190
   Thr Thr Leu Ile Ala Thr Ala Ala Phe Lys Ala Val Ala Thr Arg Leu
           195                 200                 205
   Gln Val Val Thr Pro Met Thr Pro Val Ala Val Gly Ile Asn Met Asp
       210                 215                 220
   Ser Val Gln Met Gln Val Met Asn Asp Ser Leu Lys Gly Gly Val Leu
   225                 230                 235                 240
   Tyr Cys Leu Asp Tyr Ser Lys Trp Asp Ser Thr Gln Asn Pro Ala Val
                   245                 250                 255
   Thr Ala Ala Ser Leu Ala Ile Leu Glu Arg Phe Ala Glu Pro His Pro
               260                 265                 270
   Ile Val Ser Cys Ala Ile Glu Ala Leu Ser Ser Pro Ala Glu Gly Tyr
           275                 280                 285
   Val Asn Asp Ile Lys Phe Val Thr Arg Gly Gly Leu Pro Ser Gly Met
       290                 295                 300
   Pro Phe Thr Ser Val Val Asn Ser Ile Asn His Met Ile Tyr Val Ala
   305                 310                 315                 320
   Ala Ala Ile Leu Gln Ala Tyr Glu Ser His Asn Val Pro Tyr Thr Gly
                   325                 330                 335
   Asn Val Phe Gln Val Glu Thr Val His Thr Tyr Gly Asp Asp Cys Met
               340                 345                 350
   Tyr Ser Val Cys Pro Ala Thr Ala Ser Ile Phe His Ala Val Leu Ala
           355                 360                 365
   Asn Leu Thr Ser Tyr Gly Leu Lys Pro Thr Ala Ala Asp Lys Ser Asp
       370                 375                 380
   Ala Ile Lys Pro Thr Asn Thr Pro Val Phe Leu Lys Arg Thr Phe Thr
   385                 390                 395                 400
   Gln Thr Pro His Gly Val Arg Ala Leu Leu Asp Ile Thr Ser Ile Thr
                   405                 410                 415
   Arg Gln Phe Tyr Trp Leu Lys Ala Asn Arg Thr Ser Asp Pro Ser Ser
               420                 425                 430
   Pro Pro Ala Phe Asp Arg Gln Ala Arg Ser Ala Gln Leu Glu Asn Ala
           435                 440                 445
   Leu Ala Tyr Ala Ser Gln His Gly Pro Val Val Phe Asp Thr Val Arg
       450                 455                 460
   Gln Ile Ala Ile Lys Thr Ala Gln Gly Glu Gly Leu Val Leu Val Asn
   465                 470                 475                 480
   Thr Asn Tyr Asp Gln Ala Leu Ala Thr Tyr Asn Ala Trp Phe Ile Gly
                   485                 490                 495
   Gly Thr Val Pro Asp Pro Val Gly His Thr Glu Gly Thr His Lys Ile
               500                 505                 510
   Val Phe Glu Met Glu His His His His His His
           515                 520
<210> 6
   <211> 539
   <212> PRT
   <213> Artificial
<220>
   <223> vesivirus-RdRP mit einem Histidin-Marker
<400> 6
   Met Lys Val Thr Thr Gln Lys Tyr Asp Val Thr Lys Pro Asp Ile Ser
   1 5 10 15
   Tyr Lys Gly Leu Ile Cys Lys Gln Leu Asp Glu Ile Arg Val Ile Pro
   20 25 30
   Lys Gly Thr Arg Leu His Val Ser Pro Ala His Thr Asp Asp Tyr Asp
   35 40 45
   Glu Cys Ser His Gln Pro Ala Ser Leu Gly Ser Gly Asp Pro Arg Cys
   50 55 60
   Pro Lys Ser Leu Thr Ala Ile Val Val Asp Ser Leu Lys Pro Tyr Cys
   65 70 75 80
   Glu Lys Thr Asp Gly Pro Pro His Asp Ile Leu His Arg Val Gln Arg
   85 90 95
   Met Leu Ile Asp His Leu Ser Gly Phe Val Pro Met Asn Ile Ser Ser
   100 105 110
   Glu Pro Ser Met Leu Ala Ala Phe His Lys Leu Asn His Asp Thr Ser
   115 120 125
   Cys Gly Pro Tyr Leu Gly Gly Arg Lys Lys Asp His Met Ile Gly Gly
   130 135 140
   Glu Pro Asp Lys Pro Leu Leu Asp Leu Leu Ser Ser Lys Trp Lys Leu
   145 150 155 160
   Ala Thr Gln Gly Ile Gly Leu Pro His Glu Tyr Thr Ile Gly Leu Lys
   165 170 175
   Asp Glu Leu Arg Pro Val Glu Lys Val Gln Glu Gly Lys Arg Arg Met
   180 185 190
   Ile Trp Gly Cys Asp Val Gly Val Ala Thr Val Cys Ala Ala Ala Phe
   195 200 205
   Lys Gly Val Ser Asp Ala Ile Thr Ala Asn His Gln Tyr Gly Pro Val
   210 215 220
   Gln Val Gly Ile Asn Met Asp Gly Pro Ser Val Glu Ala Leu Tyr Gln
   225 230 235 240
   Arg Ile Arg Ser Ala Ala Lys Val Phe Ala Val Asp Tyr Ser Lys Trp
   245 250 255
   Asp Ser Thr Gln Ser Pro Arg Val Ser Ala Ala Ser Ile Asp Ile Leu
   260 265 270
   Arg Tyr Phe Ser Asp Arg Ser Pro Ile Val Asp Ser Ala Ala Asn Thr
   275 280 285
   Leu Lys Ser Pro Pro Ile Ala Ile Phe Asn Gly Val Ala Val Lys Val
   290 295 300
   Thr Ser Gly Leu Pro Ser Gly Met Pro Leu Thr Ser Val Ile Asn Ser
   305 310 315 320
   Leu Asn His Cys Leu Tyr Val Gly Cys Ala Ile Leu Gln Ser Leu Glu
   325 330 335
   Ser Arg Asn Ile Pro Val Thr Trp Asn Leu Phe Ser Thr Phe Asp Met
   340 345 350
   Met Thr Tyr Gly Asp Asp Gly Val Tyr Met Phe Pro Met Met Phe Ala
   355 360 365
   Ser Val Ser Asp Gln Ile Phe Ala Asn Leu Thr Ala Tyr Gly Leu Lys
   370 375 380
   Pro Thr Arg Val Asp Lys Ser Val Gly Ala Ile Glu Pro Ile Asp Pro
   385 390 395 400
   Glu Ser Val Val Phe Leu Lys Arg Thr Ile Thr Arg Thr Pro His Gly
   405 410 415
   Ile Arg Gly Leu Leu Asp Arg Gly Ser Ile Ile Arg Gln Phe Tyr Tyr
   420 425 430
   Ile Lys Gly Glu Asn Ser Asp Asp Trp Lys Thr Pro Pro Lys Thr Ile
   435 440 445
   Asp Pro Thr Ser Arg Gly Gln Gln Leu Trp Asn Ala Cys Leu Tyr Ala
   450 455 460
   Ser Gln His Gly Pro Glu Phe Tyr Asn Lys Val Tyr Arg Leu Ala Glu
   465 470 475 480
   Lys Ala Val Glu Tyr Glu Glu Leu His Phe Glu Pro Pro Ser Tyr His
   485 490 495
   Ser Ala Leu Glu His Tyr Asn Asn Gln Phe Asn Gly Val Asp Thr Arg
   500 505 510
   Ser Asp Gln Ile Asp Ala Ser Val Met Thr Asp Leu His Cys Asp Val
   515 520 525
   Phe Glu Val Leu Glu His His His His His His
   530 535